# EUROPEAN PATENT APPLICATION

(11) **EP 4 400 149 A1**
(43) Date of publication of application: **17.07.2024**
(21) Application number: 22867130.1
(22) Date of filing: 08.08.2022
(51) Int. Cl.: A61M 25/02, A61M 39/06

(54) **OPENING/CLOSING MECHANISM FOR HEMOSTASIS VALVE, FIXING MECHANISM FOR LONG MEDICAL DEVICE, AND MEDICAL CONNECTOR**

(30) Priority: 09.09.2021 JP 2021146545
(71) Applicant: Asahi Intecc Co., Ltd., Seto-shi, Aichi 489-0071 (JP)
(72) Inventor: OSHIMIZU Ryuya, Seto-shi, Aichi 489-0071 (JP)
(74) Representative: Winter, Brandl - Partnerschaft mbB
(86) International application number: PCT/JP2022/030210
(87) International publication number: WO 2023/037808

(57) **Abstract**

An opening/closing mechanism comprises a housing, a hemostasis valve, a through-member, an operating member, a force transmission mechanism, and a holding mechanism. The through-member can be positioned along a first direction in a first position that places the hemostasis valve in a closed state, and a second position that presses the hemostasis valve and places the hemostasis valve in an open state. The operating member can slide along a second direction not parallel to the first direction. The force transmission member can be positioned in a third position that positions the through-member in the first position, and a fourth position that presses the through-member and positions the through-member in the second position. The holding mechanism switches to a state where the force transmission member is held in the third position, or held in the fourth position each time the operating member slides along the second direction.

## Description

### TECHNICAL FIELD

The technique disclosed herein relates to an opening/closing mechanism for a hemostasis valve, a fixing mechanism for a long medical device, and a medical connector.

### BACKGROUND ART

A Y connector is a medical connector that is used by being connected to a guiding catheter. A Y connector has a main pipe portion and a branched pipe portion that is branched from the main pipe portion, where a long medical device such as a guide wire or a catheter is introduced into the guiding catheter via the main pipe portion, and a liquid agent such as a contrast medium or physiological saline is provided via the branched pipe portion. AY connector is provided with an opening/closing mechanism for opening and closing a hemostasis valve that suppresses the outflow of blood via the lumen of the main pipe portion, and a fixing mechanism for fixing a long medical device.

An opening/closing mechanism for a hemostasis valve of a conventional Y connector is provided with a through-member (opener) formed having a through-hole that is coaxial with the lumen of the main pipe portion, and as a result of performing an operation of pressing the through-member in a direction parallel to the axial direction of the lumen of the main pipe portion, it is possible to switch between an open state in which the through-member presses the hemostasis valve and opens the hemostasis valve, and a closed state in which the through-member separates from the hemostasis valve and closes the hemostasis valve (for example, see Patent Literature 1). Furthermore, the fixing mechanism for a long medical device in the conventional Y connector is provided with an elastic fixing valve formed having a through-hole that the long medical device is inserted through, and as a result of performing a rotation operation with respect to a screw to move the screw in the axial direction, which accordingly moves a pusher in the axial direction, it is possible to switch between a fixed state, in which the fixing valve undergoes elastic deformation as a result of being pressed by the pusher, which causes a reduction in the inner diameter of the through-hole of the fixing valve and causes the long medical device to be fixed, and an unfixed state in which the fixing valve is not pressed by the pusher, which results in an expansion in the inner diameter of the through-hole of the fixing valve and the release of the fixing of the long medical device (for example, see Patent Literature 1).

### CITATION LIST

### Patent Literature

Patent Literature 1: Japanese Patent No. 5249049

### SUMMARY OF INVENTION

### TECHNICAL PROBLEM

In the configuration of the conventional Y connector described above, because the opening and closing operation of the hemostasis valve is an operation of pressing the through-member in the axial direction of the lumen of the main pipe portion, there is a problem that the operation is difficult to perform. In addition, in the configuration of the conventional Y connector described above, because the fixing/unfixing operation of the long medical device is an operation that causes the screw to rotate, there is a problem that the operation is complicated, and it is not possible to grasp, visually or by feel, the degree to which the long medical device is fixed.

A technique capable of solving the problems described above is disclosed herein.

### SOLUTION TO PROBLEM

The technique disclosed herein can be achieved, for example, as the following aspects.

(1) An opening/closing mechanism for a hemostasis valve disclosed herein comprises a housing, a hemostasis valve, a through-member, an operating member, a force transmission member, and a holding mechanism. The housing is a tubular member formed having a lumen that communicates with a distal end side opening and a proximal end side opening. The hemostasis valve is attached inside the housing and is normally in a closed state, and is switched to an open state when pressed from a proximal end side such that a through-hole is formed that communicates with the distal end side opening of the housing. The through-member is accommodated inside the housing so as to be capable of sliding along a first direction, being an extending direction of the lumen on a proximal end side of the hemostasis valve. The through-member is formed having a through-hole that communicates with the proximal end side opening of the housing. The through-member is capable of being positioned in a first position that places the hemostasis valve in the closed state, and a second position, being a position further toward a distal end side along the first direction than the first position, which places the hemostasis valve in the open state by pressing the hemostasis valve, and communicates the through-hole of the hemostasis valve and the through-hole of the through-member. The operating member is a member that is capable of sliding along a second direction that is not parallel to the first direction. The force transmission member is disposed so as to be capable of sliding in the second direction between the through-member and the operating member, and is a member that transmits, to the through-member, a force that moves the operating member along the second direction so as to approach the through-member. The force transmission member is capable of being positioned in a third position that places the through-member in the first position, and a fourth position that presses the through-member and places the through-member in the second position. The holding mechanism is a mechanism that switches to a state in which the force transmission member is held in the third position, or a state in which the force transmission member is held in the fourth position each time the operating member slides along the second direction.
   In this way, in this opening/closing mechanism, the holding mechanism switches to a state in which the force transmission member is held in the third position, or a state in which the force transmission member is held in the fourth position each time the operating member slides along the second direction. When the holding mechanism switches with the sliding of the operating member to a state in which the force transmission member is held in the fourth position, the through-member moves from the first position to the second position, and the hemostasis valve switches from the closed state to the open state. At this time, because the holding mechanism holds the force transmission member in the fourth position, the open state of the hemostasis valve is maintained. Furthermore, when the holding mechanism switches with the sliding of the operating member once again to a state in which the force transmission member is held in the third position, the through-member moves from the second position to the first position, and the hemostasis valve switches from the open state to the closed state. At this time, because the holding mechanism holds the force transmission member in the third position, the closed state of the hemostasis valve is maintained. Moreover, in this opening/closing mechanism, because the operating member is capable of sliding along the second direction, which is not parallel to the extending direction of the lumen of the housing, the opening and closing operation of the hemostasis valve becomes an operation of pressing the operating member in the second direction, which is not parallel to the extending direction of the lumen of the housing. As a result, according to this opening/closing mechanism, it is possible for an operator to easily perform the opening and closing operation of the hemostasis valve with the thumb or index finger while gripping a device to which this opening/closing mechanism is provided, and the operability of the opening/closing mechanism can be improved.
(2) In the opening/closing mechanism for a hemostasis valve above, the operating member and the force transmission member may be an integrated member. According to this opening/closing mechanism for a hemostasis valve, the number of components can be reduced.
(3) In the opening/closing mechanism for a hemostasis valve above, the holding mechanism may include a cylindrical biasing member that directly biases, or biases via another member, the force transmission member along the second direction toward an external side of the housing, and a surface of the force transmission member or of the another member facing the biasing member may be formed having a protrusion that is inserted into a hollow portion of the biasing member. According to this opening/closing mechanism for a hemostasis valve, it is possible to easily and accurately perform the positioning between the force transmission member and the biasing member, and it is also possible to effectively transmit the biasing force of the biasing member to the force transmission member, which enables the accuracy of the motion of the opening/closing mechanism to be improved.
(4) In the opening/closing mechanism for a hemostasis valve above, an angle formed between the first direction and the second direction may be 35 degrees or more and 55 degrees or less. According to this opening/closing mechanism for a hemostasis valve, it is possible for an operator to very easily perform the opening and closing operation of the hemostasis valve with the thumb while gripping a device to which this opening/closing mechanism is provided, and the operability of the opening/closing mechanism can be effectively improved.
(5) In the opening/closing mechanism for a hemostasis valve above, the through-member may include a main body portion that is formed having a through-hole, and a flange portion that protrudes from the main body portion in a third direction, which is orthogonal to the first direction, and the force transmission member may make contact with a surface of the flange portion on a proximal end side, while also being configured so as to be capable of a relative sliding movement with respect to the through-member along the third direction. According to this opening/closing mechanism for a hemostasis valve, the sliding of the force transmission member along the second direction that is not parallel to the extending direction of the lumen of the housing and the sliding of the through-member in the extending direction can be efficiently converted, and the operability of the opening/closing mechanism can be effectively improved.
(6) In the opening/closing mechanism for a hemostasis valve above, an angle formed between the first direction and the second direction may be substantially 90 degrees. According to this opening/closing mechanism for a hemostasis valve, it is possible for an operator to very easily perform the opening and closing operation of the hemostasis valve with the thumb or index finger while gripping a device to which this opening/closing mechanism is provided, and the operability of the opening/closing mechanism can be effectively improved.
(7) In the opening/closing mechanism for a hemostasis valve above, the force transmission member may be capable of sliding in the second direction, the through-member may include a protrusion that protrudes in a fourth direction, which is orthogonal to both the first direction and the second direction, and the force transmission member may be formed having a groove into which the protrusion is inserted, and which extends in a direction that is orthogonal to the fourth direction and not parallel to both the first direction and the second direction. According to this opening/closing mechanism for a hemostasis valve, the sliding of the force transmission member along the second direction, which is substantially orthogonal to the extending direction of the lumen of the housing, and the sliding of the through-member in the extending direction can be efficiently converted, and the operability of the opening/closing mechanism can be effectively improved.
(8) The fixing mechanism for a long medical device disclosed herein includes a housing, a cylindrical body, a pressing member, an operating member, a force transmission member, and a holding mechanism. The housing is a tubular member formed having a lumen that communicates with a distal end side opening and a proximal end side opening. The cylindrical body is attached inside the housing, and is a flexible member formed having a through-hole into which the long medical device is inserted. The through-hole of the cylindrical body communicates with the distal end side opening and the proximal end side opening of the housing. The pressing member is accommodated inside the housing and is capable of sliding along a sixth direction, which is a direction orthogonal to a fifth direction that is parallel to an axis of the cylindrical body. The pressing member is capable of being positioned in a fifth position, and a sixth position that is displaced from the fifth position along the sixth direction, at which a part of the cylindrical body excluding both end portions is deformed by being pressed from an outer peripheral side. The operating member is a member that is capable of sliding along a seventh direction that is not parallel to the fifth direction. The force transmission member is disposed so as to be capable of sliding in the seventh direction between the pressing member and the operating member, and is a member that transmits, to the pressing member, a force that moves the operating member along the seventh direction so as to approach the pressing member. The force transmission member is capable of being positioned in a seventh position that places the pressing member in the fifth position, and an eighth position that presses the pressing member and positions the pressing member in the sixth position. The holding mechanism switches to a state in which the force transmission member is held in the seventh position, or a state in which the force transmission member is held in the eighth position each time the operating member slides along the seventh direction. This fixing mechanism for a long medical device is configured such that, when the pressing member is positioned in the sixth position, an inner peripheral surface of the deformed cylindrical body is pressed against the long medical device, which causes the long medical device to become fixed by the cylindrical body.
   In this way, in this fixing mechanism for a long medical device, the holding mechanism switches to a state in which the force transmission member is held in the seventh position, or a state in which the force transmission member is held in the eighth position each time the operating member slides along the seventh direction. When the holding mechanism switches with the sliding of the operating member to a state in which the force transmission member is held in the eighth position, the pressing member moves from the fifth position to the sixth position, and the cylindrical body undergoes elastic deformation and the long medical device is fixed by the cylindrical body. At this time, because the holding mechanism holds the force transmission member in the eighth position, the fixed state of the long medical device is maintained. Furthermore, when the holding mechanism switches with the sliding of the operating member again to a state in which the force transmission member is held in the seventh position, the pressing member moves from the sixth position to the fifth position, and shape of the cylindrical body is restored which releases the fixing of the long medical device. At this time, because the holding mechanism holds the force transmission member in the seventh position, a state in which the fixing of the long medical device has been released is maintained. Moreover, in this fixing mechanism for a long medical device, because the operating member is capable of sliding along the seventh direction, which is not parallel to a direction parallel to an axis of the cylindrical body, the fixing operation and unfixing operation of the long medical device becomes an operation of pressing the operating member in the seventh direction, which is not parallel to a direction parallel to an axis of the cylindrical body. Therefore, according to this fixing mechanism for a long medical device, it is possible to achieve the fixing and unfixing of the long medical device with a simple operation in which the operator presses the operating member with the thumb while gripping a device provided with this fixing mechanism, and because the degree to which the long medical device is fixed can be grasped visually or by feel, the operability of the fixing mechanism can be improved.
(9) In the fixing mechanism for a long medical device above, the holding mechanism may include a cylindrical biasing member that biases the force transmission member in a direction approaching the pressing member, and a surface of the force transmission member facing the biasing member may be formed having a protrusion that is inserted into a hollow portion of the biasing member. According to this fixing mechanism for a long medical device, it is possible to easily and accurately perform the positioning between the force transmission member and the biasing member, and it is also possible to effectively transmit the biasing force of the biasing member to the force transmission member, which enables the accuracy of the motion of the fixing mechanism to be improved.
(10) In the fixing mechanism for a long medical device above, an angle formed between the fifth direction and the seventh direction may be 35 degrees or more and 55 degrees or less. According to this fixing mechanism for a long medical device, it is possible to very easily operate the operating member with the thumb while gripping a device to which this fixing mechanism is provided, and the operability of the fixing mechanism can be effectively improved.
(11) In the fixing mechanism for a long medical device above, the force transmission member may make contact with a surface of the pressing member, while also being configured to be capable of a relative sliding movement with respect to the pressing member along the fifth direction. According to this fixing mechanism for a long medical device, the sliding of the force transmission member along the seventh direction and the sliding of the pressing member along the seventh direction can be efficiently converted, and the operability of the fixing mechanism can be effectively improved.
(12) Another fixing mechanism for a long medical device disclosed herein includes a housing, a cylindrical body, a distal end side connection portion, a proximal end side connection portion, and a pressing member. The housing is a tubular member formed having a lumen that communicates with a distal end side opening and a proximal end side opening, and which is capable of accommodating the long medical device in the lumen. The flexible cylindrical body is attached inside the housing and is formed having a first through-hole into which the long medical device is inserted, the first through-hole communicating with the distal end side opening and the proximal end side opening of the housing. The distal end side connection portion connects a distal end portion of the cylindrical body to the housing, and is formed having a second through-hole that communicates with the first through-hole of the cylindrical body. The proximal end side connection portion connects a proximal end portion of the cylindrical body to the housing, and is formed having a third through-hole that communicates with the first through-hole of the cylindrical body. The pressing member presses a pressed part of the cylindrical body excluding both end portions from a direction that is not parallel to an axis of the cylindrical body, and is accommodated inside the housing. An area of the second through-hole of the distal end side connection portion in a transverse cross-section of the fixing mechanism and an area of the third through-hole in the transverse cross-section of the proximal end side connection portion are larger than an area of the first through-hole of the pressed part in the transverse cross-section.

In this way, in this fixing mechanism for a long medical device, when the pressing member presses the pressed part of the cylindrical body excluding both end portions, the cylindrical body undergoes elastic deformation and the long medical device is fixed by the cylindrical body. At this time, because the area of the second through-hole of the distal end side connection portion and the area of the third through-hole of the proximal end side connection portion are larger than the area of the first through-hole of the pressed part of the cylindrical body, even when the long medical device is fixed as a result of the pressing member pressing the pressed part of the cylindrical body, the long medical device is prevented from being pressed against the inner peripheral surface of the distal end side connection portion and the proximal end side connection portion, and damage to the long medical device can be more effectively suppressed.

The technique disclosed in herein can be realized in various forms, and can be realized in a form such as an opening/closing mechanism for a hemostasis valve, a fixing mechanism for a long medical device, a medical connector provided with an opening/closing mechanism for a hemostasis valve and/or a fixing mechanism for a long medical device, or a medical device provided with a medical connector.

### BRIEF DESCRIPTION OF DRAWINGS

FIG. 1 is an explanatory diagram showing a configuration of a medical connector according to a first embodiment.
FIG. 2 is an explanatory diagram showing a configuration of a medical connector.
FIG. 3 is an explanatory diagram showing a configuration of an opening/closing mechanism of a medical connector.
FIG. 4 is an explanatory diagram showing a configuration of the opening/closing mechanism shown in FIG. 3.
FIG. 5 is an explanatory diagram showing a configuration of an opening/closing mechanism.
FIG. 6 is a cross-sectional perspective view showing a configuration of a proximal end side housing part that constitutes a housing of an opening/closing mechanism.
FIG. 7 is a perspective view showing an external configuration of a through-member and a force transmission member of an opening/closing mechanism.
FIG. 8 is a perspective view showing an external configuration of an operating member and a holding mechanism of an opening/closing mechanism.
FIG. 9 is a perspective view showing an external configuration of an outer cylinder of an opening/closing mechanism.
FIG. 10 is an explanatory diagram showing a configuration of a modification of the opening/closing mechanism of the first embodiment.
FIG. 11 is an explanatory diagram showing a configuration of a fixing mechanism according to the first embodiment.
FIG. 12 is an explanatory diagram showing a configuration of a fixing mechanism.
FIG. 13 is a cross-sectional perspective view showing a configuration of a cylindrical body, a pressing member, and a force transmission member of a fixing mechanism.
FIG. 14 is an explanatory diagram showing an external configuration of a medical connector according to a second embodiment.
FIG. 15 is an explanatory diagram showing a configuration of an opening/closing mechanism of the medical connector shown in FIG. 14.
FIG. 16 is an explanatory diagram showing a configuration of the opening/closing mechanism shown in FIG. 15.
FIG. 17 is an explanatory diagram showing a configuration of an opening/closing mechanism.
FIG. 18 is a cross-sectional perspective view showing a configuration of a housing of an opening/closing mechanism.
FIG. 19 is a perspective view showing an external configuration of a through-member and a force transmission member of an opening/closing mechanism.
FIG. 20 is an explanatory diagram showing a configuration of a modification of the opening/closing mechanism of the second embodiment.
FIG. 21 is an explanatory diagram showing a configuration of a longitudinal cross-section of a medical connector according to a third embodiment.
FIG. 22 is an explanatory diagram showing a state of the fixing mechanism shown in

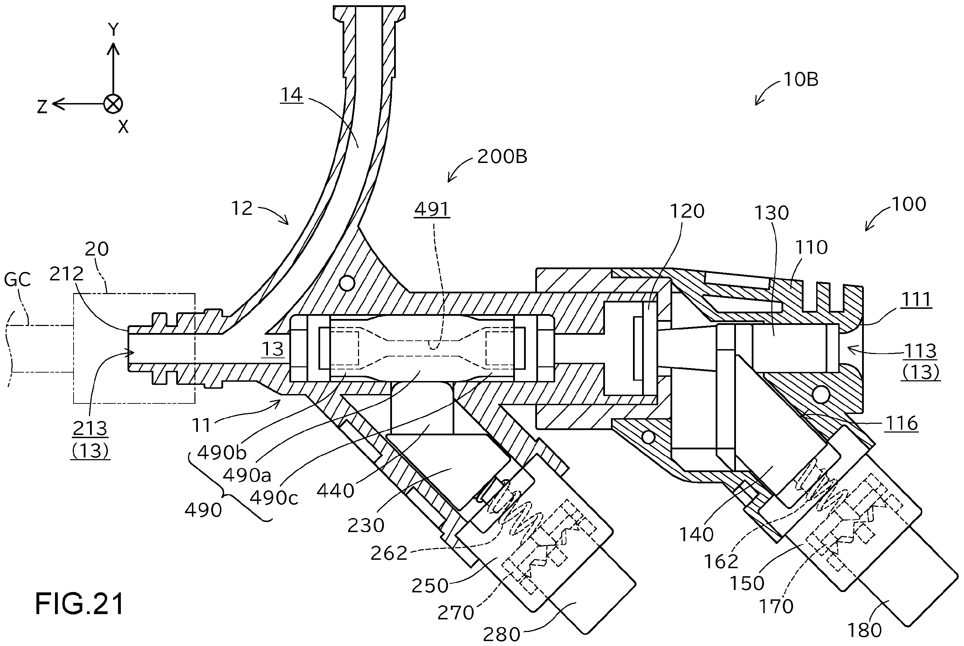

FIG. 21 where fixing of a long medical device has been released.

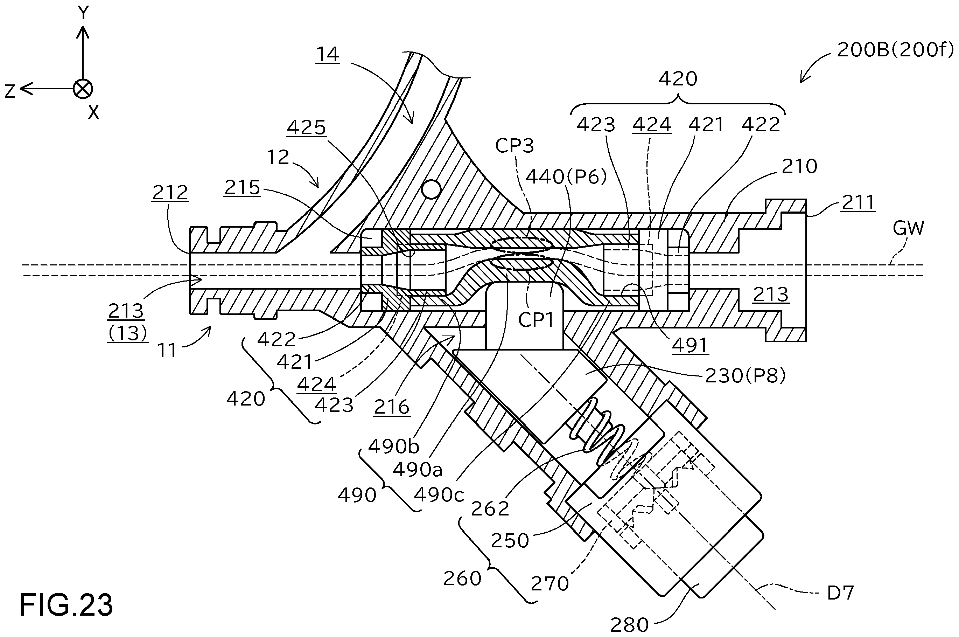

FIG. 23 is an explanatory diagram showing a state of the fixing mechanism shown in
FIG. 21 where a long medical device has been fixed.

### EMBODIMENTS OF THE INVENTION

### A. First Embodiment:

### A-1. Configuration of Medical Connector:

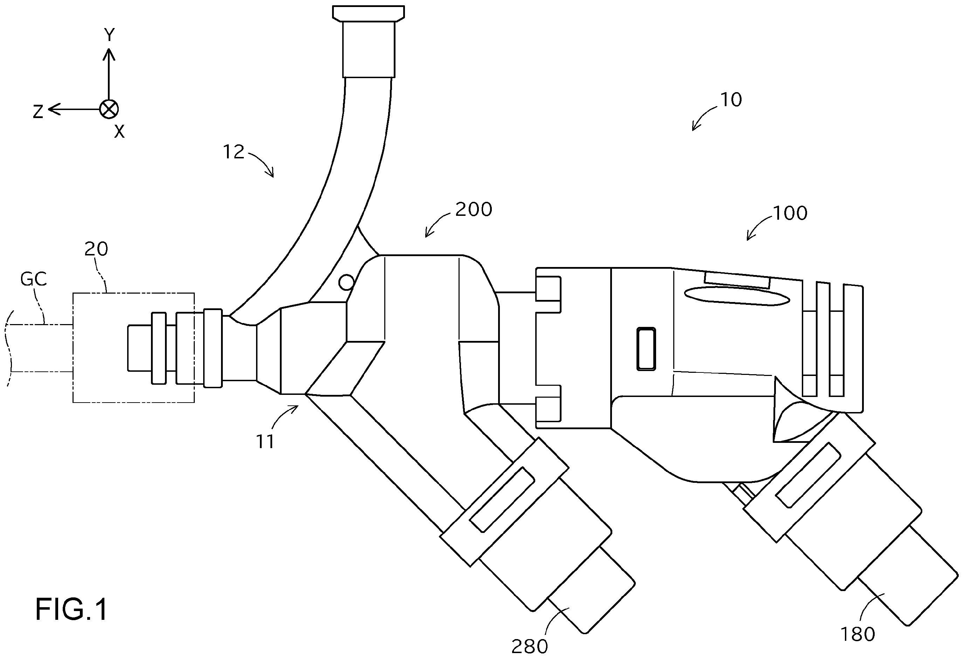

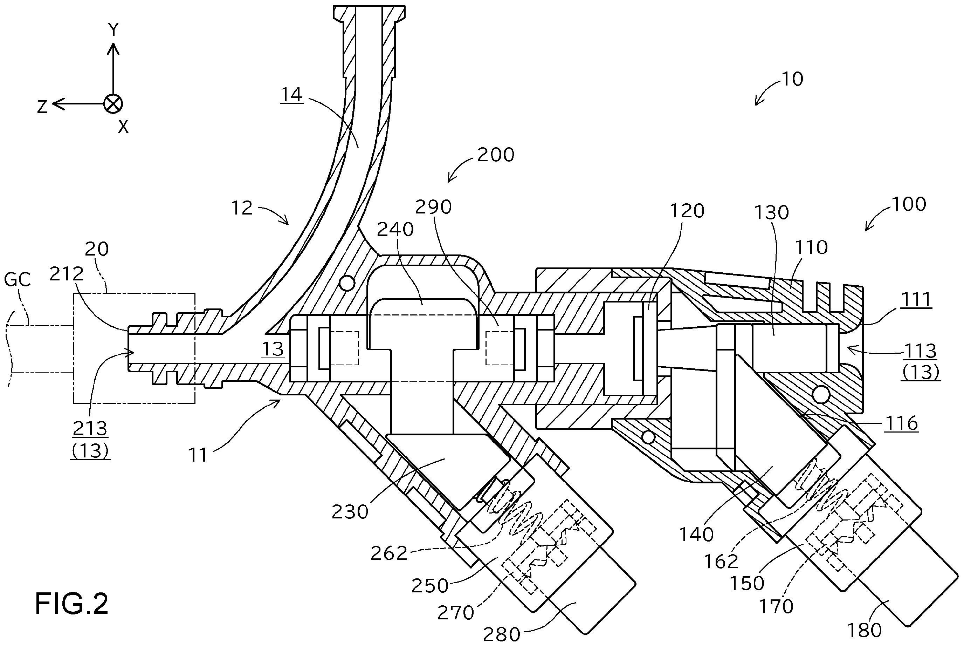

FIGS. 1 and 2 are explanatory diagrams showing a configuration of a medical connector in a first embodiment. FIG. 1 schematically shows an external configuration of a medical connector 10, and FIG. 2 shows a longitudinal cross-section (YZ cross-section) of the medical connector 10. The medical connector 10 is a Y connector that is used by being connected to a guiding catheter GC via a rotator 20. Herein, the side of the medical connector 10 to which the guiding catheter GC is connected (positive Z-axis direction side) is referred to as the distal end side, and the opposite side (negative Z-axis direction side) is referred to as the proximal end side. Furthermore, in the medical connector 10 and each of the constituent members, the end on the distal end side is referred to as the "distal end", the distal end and the vicinity thereof are referred to as the "distal end portion", the end on the proximal end side is referred to as the "proximal end", and the proximal end and the vicinity thereof are referred to as the "proximal end portion". Moreover, for convenience of description, the Z-axis direction is also referred to as the front-rear direction, the Y-axis direction is also referred to as the up-down direction, the positive Y-axis direction is also referred to as the upward direction, the negative Y-axis direction is also referred to as the downward direction, and the X-direction is also referred to as the left-right direction. However, the posture of the medical connector 10 is not limited to this. Further, in the following cross-sectional views, the configuration of the side surfaces of some members is omitted in some cases.

The medical connector 10 includes a tubular main pipe portion 11 extending in the front-rear direction, and a tubular branched pipe portion 12 branching from the vicinity of a distal end portion of the main pipe portion 11 and diagonally extends toward an upper proximal end side. The main pipe portion 11 is formed having a lumen 13 that extends in the front-rear direction and passes through the main pipe portion 11, and a long medical device (not illustrated) such as a guide wire or a catheter is introduced into the guiding catheter GC via the lumen 13. Furthermore, the branched pipe portion 12 is formed having a lumen 14 that communicates with the lumen 13 of the main pipe portion 11, and a liquid agent such as a contrast medium or physiological saline is provided via the lumen 14 from a liquid agent supply apparatus (not illustrated) connected to an end portion of the branched pipe portion 12.

The medical connector 10 includes an opening/closing mechanism 100 for a hemostasis valve, and a fixing mechanism 200 for a long medical device. The opening/closing mechanism 100 is provided further toward the proximal end side than the fixing mechanism 200. The opening/closing mechanism 100 constitutes a portion of the proximal end side of the main pipe portion 11 of the medical connector 10, and the fixing mechanism 200 constitutes a portion of the distal end side of the main pipe portion 11 of the medical connector 10 and the branched pipe portion 12. Hereinafter, the configurations of the opening/closing mechanism 100 and the fixing mechanism 200 will be described in turn.

The medical connector 10 is used by being gripped by an operator such as a physician. For example, the operator grips the main pipe portion 11 of the medical connector 10 in the posture shown in FIG. 1 with four fingers from the index finger to the little finger, and grasps the medical connector 10 such that the thumb of the operator is positioned in the vicinity of an operating member 180 of the opening/closing mechanism 100 or an operating member 280 of the fixing mechanism 200.

### A-2. Configuration of Opening/Closing Mechanism:

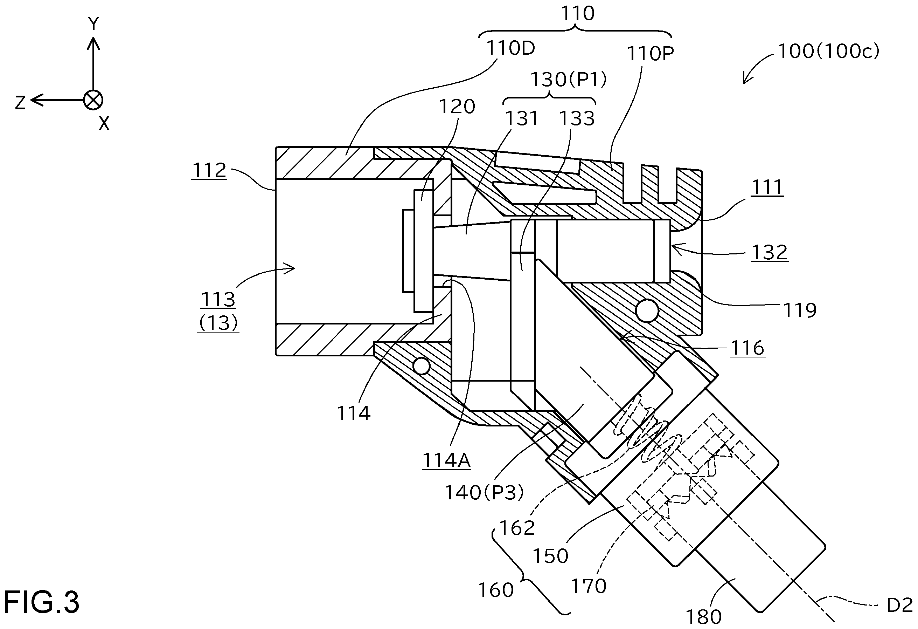

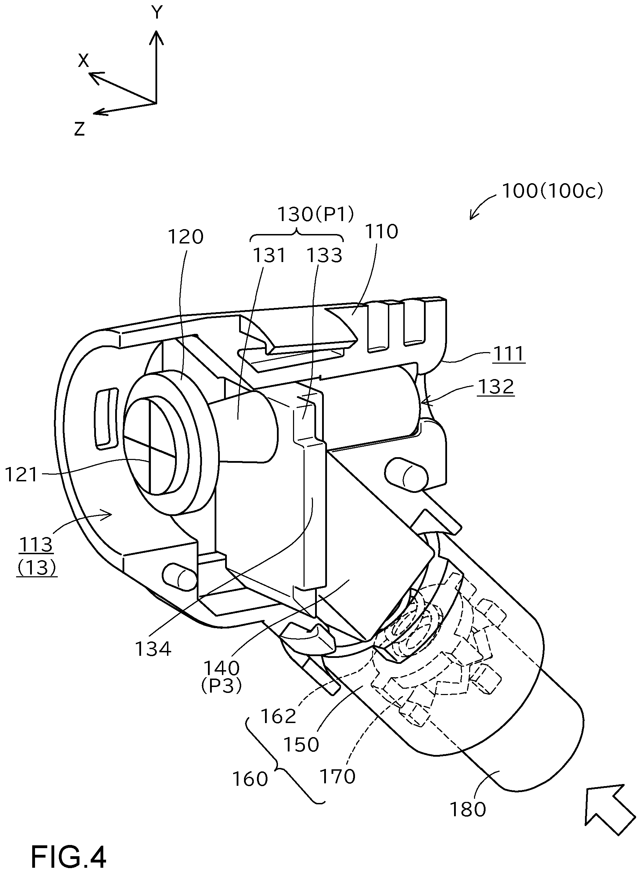

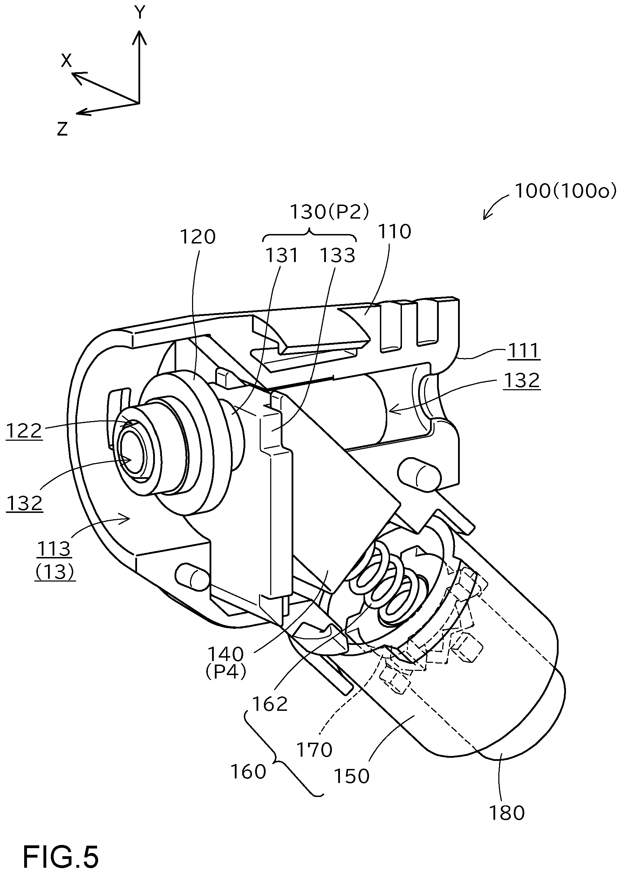

Next, the configuration of the opening/closing mechanism 100 for a hemostasis valve 120 will be described. FIGS. 3 to 5 are explanatory diagrams showing the configuration of the opening/closing mechanism 100 in the first embodiment. FIG. 3 shows a longitudinal cross-section (YZ cross-section), and FIGS. 4 and 5 show perspective views. FIGS. 3 and 4 show the opening/closing mechanism 100 in a state in which the hemostasis valve 120 is closed (hereinafter referred to as "closed state opening/closing mechanism 100c"), and FIG. 5 shows the opening/closing mechanism 100 in a state in which the hemostasis valve 120 is open (hereinafter referred to as "open state opening/closing mechanism 100o).

The opening/closing mechanism 100 is a mechanism for opening and closing the hemostasis valve 120, which suppresses the outflow of blood via the lumen 13 of the main pipe portion 11 (FIG. 2) of the medical connector 10. The opening/closing mechanism 100 is a mechanism that switches the hemostasis valve 120 to the closed state or the open state each time the operator performs a pressing operation with respect to the operating member 180. The opening/closing mechanism 100 includes a housing 110, a hemostasis valve 120, a through-member 130, an operating member 180, a force transmission member 140, and a holding mechanism 160.

The housing 110 is formed having a distal end side opening 112 and a proximal end side opening 111, and is a tubular member formed having a lumen 113 that communicates with the distal end side opening 112 and the proximal end side opening 111. The lumen 113 is a through-hole extending in the front-rear direction (Z-axis direction), and constitutes a portion of the lumen 13 of the main pipe portion 11 of the medical connector 10. The housing 110, for example, is formed of a resin. As shown in FIG. 2, the proximal end portion of the fixing mechanism 200 is inserted and fixed inside the lumen 113. The Z-axis direction is an example of a first direction in the claims.

The housing 110 is configured by a distal end side housing part 110D and a proximal end side housing part 110P. Each of the distal end side housing part 110D and the proximal end side housing part 110P is a tubular member formed having a lumen extending in the front-rear direction. As a result of the proximal end portion of the distal end side housing part 110D being inserted and fixed inside the lumen of the distal end portion of the proximal end side housing part 110P, the proximal end side housing part 110P and the distal end side housing part 110D are integrated to form the housing 110. In the vicinity of the center of the inside of the housing 110 in the front-rear direction, a substantially flat plate-shaped partition wall 114 that is substantially orthogonal to the front-rear direction is formed by a wall portion at the proximal end of the distal end side housing part 110D. The partition wall 114 is formed having a through-hole 114A that passes through the partition wall 114 in the front-rear direction and constitutes a portion of the lumen 113. The transverse cross-sectional shape of the through-hole 114A, for example, is substantially circular. In FIGS. 4 and 5, illustration of the distal end side housing part 110D has been omitted.

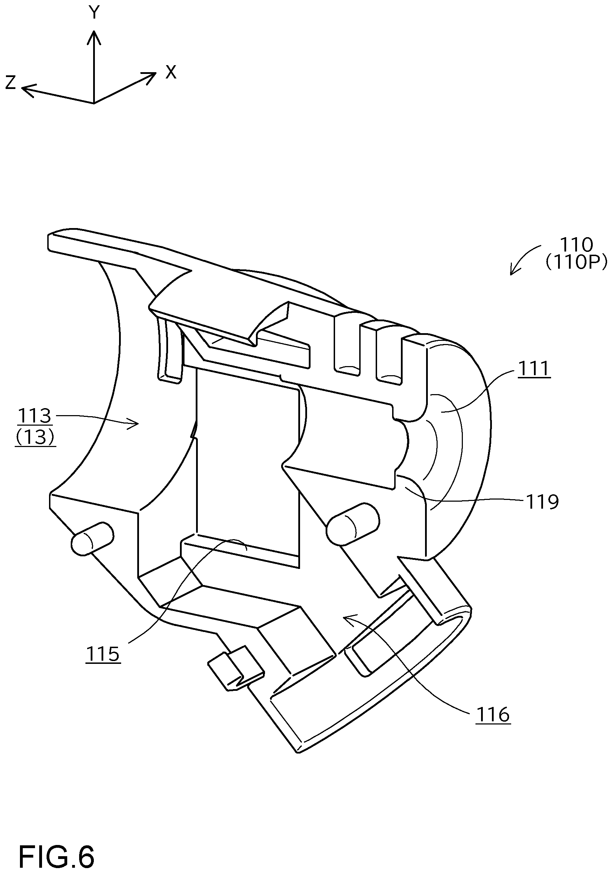

FIG. 6 is a cross-sectional perspective view showing the configuration of the proximal end side housing part 110P that constitutes the housing 110 in the first embodiment. A side surface inside the proximal end side housing part 110P is formed having guide grooves 115 that extend in the front-rear direction. The guide grooves 115 are formed as a pair that face each other in the left-right direction (X-axis direction). Furthermore, the proximal end portion inside the proximal end side housing part 110P is formed having a member accommodating space 116 that communicates with the lumen 113, and extends in a diagonal direction toward the lower proximal end side and is open at the surface of the proximal end side housing part 110P.

As shown in FIGS. 3 to 5, the hemostasis valve 120 is a substantially circular plate-shaped member, and is formed of an elastic material such as silicone rubber. The hemostasis valve 120 is fixed at a position further toward the distal end side than the partition wall 114 inside the housing 110. A slit 121 is formed at a position substantially at the center of the hemostasis valve 120 when viewed in the Z-axis direction (FIG. 4). The hemostasis valve 120 is normally in the closed state in which the slit 121 is closed and the valve is closed (FIGS. 3 and 4). When the hemostasis valve 120 is in the closed state, the lumen 113 is closed by the hemostasis valve 120, and the outflow of blood to the proximal end side of the hemostasis valve 120 via the lumen 113 is suppressed. Furthermore, when the hemostasis valve 120 is pressed from the proximal end side, each piece divided by the slit 121 elastically deforms and is displaced toward the distal end side, and the hemostasis valve 120 switches to the open state, in which a through-hole 122 is formed that passes through the hemostasis valve 120 in front-rear direction (FIG. 5). In the open state, the through-hole 122 formed in the hemostasis valve 120 communicates with the distal end side opening 112 via the lumen 113. Therefore, when the hemostasis valve 120 is in the open state, the lumen 113 is not closed at the position of the hemostasis valve 120, and is in a pass-through state. When the pressing force from the proximal end side is no longer applied, the hemostasis valve 120 is elastically deformed and returns to the closed state.

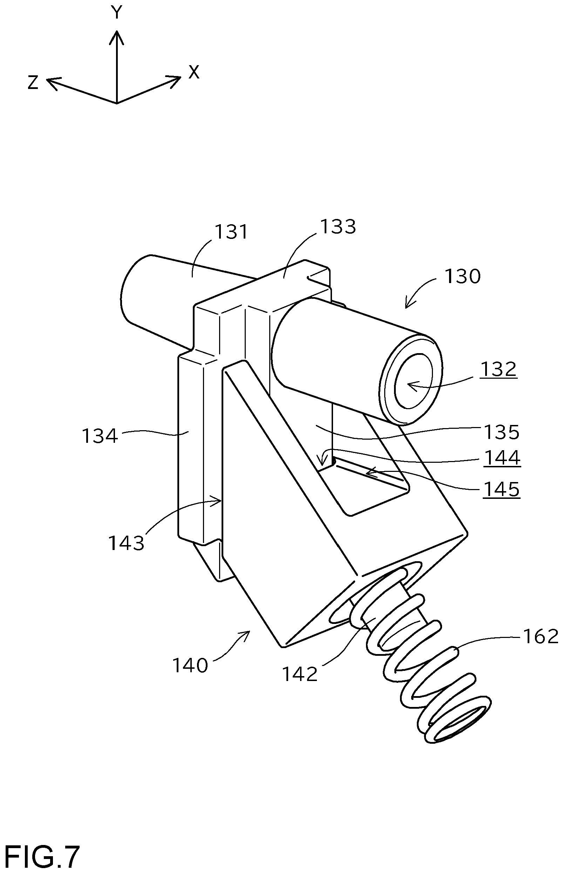

FIG. 7 is a perspective view showing an external configuration of the through-member 130 and the force transmission member 140 of the first embodiment. As shown in FIGS. 3 to 5, and FIG. 7, the through-member 130 is a member formed having a through-hole 132 extending in the front-rear direction, and for example, is formed of a resin. More specifically, the through-member 130 includes a substantially circular and tubular main body portion 131 formed having the through-hole 132 extending in the front-rear direction, and a substantially flat plate-shaped flange portion 133 that downwardly protrudes from the vicinity of the center in the front-rear direction, and is substantially orthogonal to the front-rear direction. A pair of guide protrusions 134 that protrude in the left-right direction are formed on a side surface of the flange portion 133, and a continuous guide protrusion 135 extending in the up-down direction is formed on the proximal end side surface of the flange portion 133. The up-down direction is an example of a third direction in the claims.

The through-member 130 is accommodated in the housing 110 further toward the proximal end side than the hemostasis valve 120. In the through-member 130, the through-hole 132 communicates with the proximal end side opening 111 of the housing 110. Furthermore, the through-hole 132 and the lumen 113 of the housing 110 are coaxial with each other.

In the through-member 130, the pair of guide protrusions 134 formed on the flange portion 133 (FIG. 7) are fitted into the pair of guide grooves 115 formed in the housing 110 (FIG. 6). As a result, the through-member 130 is capable of sliding in the front-rear direction with the guide grooves 115 as a guide in a state in which the position with respect to the housing 110 in the up-down direction and the left-right direction is fixed. The through-member 130 is positioned such that the main body portion 131 faces the through-hole 114A of the partition wall 114 in the front-rear direction. The through-member 130, which is capable of sliding in the front-rear direction, has the distal end portion of the main body portion 131 inserted into the through-hole 114A, and can be positioned in a pressing position P2 that presses the hemostasis valve 120 and places the hemostasis valve 120 in the open position, and a non-pressing position P1 that does not press the hemostasis valve 120 and places the hemostasis valve 120 in the closed position. As shown in FIG. 5, in the state in which the through-member 130 is positioned in the pressing position P2, the through-hole 122 formed in the hemostasis valve 120 and the through-hole 132 of the through-member 130 communicate with each other. The distal end side of the through-member 130 is capable of moving to a position in which the flange portion 133 makes contact with the partition wall 114 of the housing 110, and the proximal end side is capable of moving to a position in which the proximal end of the through-member 130 makes contact with a protrusion 119 formed on the proximal end portion of the housing 110 (FIGS. 3 and 6). The pressing position P2 of the through-member 130 is an example of a second position in the claims, and the non-pressing position P1 of the through-member 130 is an example of a first position in the claims.

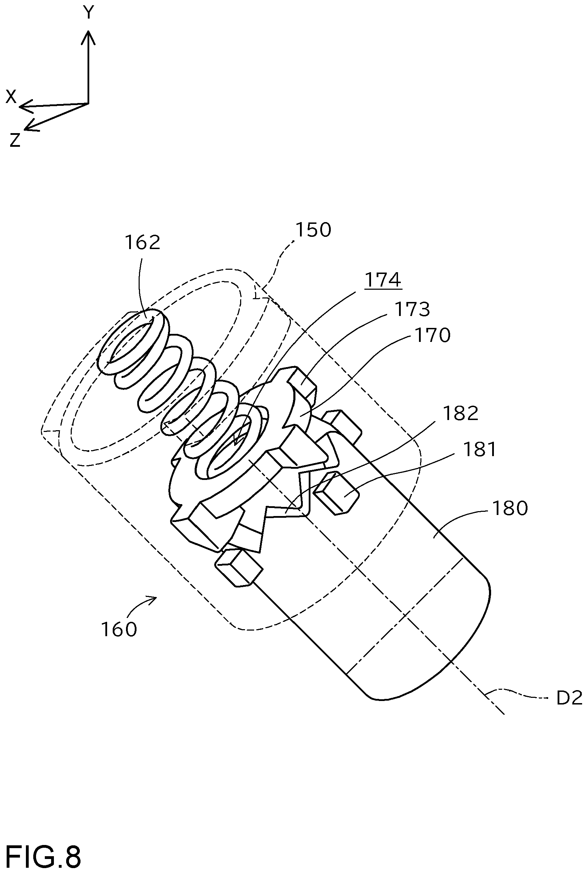

FIG. 8 is a perspective view showing an external configuration of the operating member 180 and the holding mechanism 160 of the first embodiment. As shown in FIGS. 3 to 5 and FIG. 8, the operating member 180 is a substantially hollow cylindrical member extending in a direction (hereinafter referred to as "operation direction D2") not parallel to the front-rear direction (Z-axis direction). In the present embodiment, the operation direction D2 is a direction at an angle of 35 degrees or more and 55 degrees or less with respect to the front-rear direction, and for example, is a direction at an angle of 45 degrees with respect to the front-rear direction, that is, a direction inclined at 45 degrees from the upper distal end side toward the lower proximal end side. The operating member 180 is supported by an outer cylinder 150 of the holding mechanism 160 described below, and is capable of sliding along the operation direction D2. A part of the operating member 180 on the proximal end side is exposed from the outer cylinder 150, and enables a pressing operation to be made by an operator such as a physician. The operating member 180, for example, is formed of a resin. The operation direction D2 is an example of a second direction in the claims.

The force transmission member 140 is a substantially polygonal member, and for example, is formed of a resin. A distal end surface 143 of the force transmission member 140 has a planar shape that is substantially orthogonal to the front-rear direction, and makes contact with the proximal end side surface of the flange portion 133 of the through-member 130. The distal end surface 143 is formed having a guide groove 144 that continuously extends in the up-down direction. The guide protrusion 135 of the through-member 130 is fitted into the guide groove 144. Furthermore, an upper surface of the force transmission member 140 is formed having a recess 145 that is capable of accommodating the proximal end portion of the main body portion 131 of the through-member 130. Furthermore, a surface on the lower proximal end side of the force transmission member 140 is formed having a projection-shaped protrusion 142.

The force transmission member 140 is accommodated so as to be positioned between the through-member 130 and the operating member 180 in the member accommodating space 116 of the housing 110, and is capable of sliding along the operation direction D2. The force transmission member 140 is capable of transmitting to the through-member 130 a force along the operation direction D2 that causes the operating member 180 to approach the through-member 130.

Because the force transmission member 140 has the configuration described above, it makes contact with the proximal end side surface of the flange portion 133 of the through-member 130, while also being capable of a relative sliding movement with respect to the through-member 130 along the up-down direction. That is, as shown in FIGS. 3 and 4, when the through-member 130 is in the non-pressing position P1 where it does not press the hemostasis valve 120, the force transmission member 140 is in a retracted position P3 where it is retracted to the lower proximal end side. When the force transmission member 140 is in the retracted position P3, the force transmission member 140 does not press the through-member 130. When the through-member 130 is not being pressed by the force transmission member 140, the through-member 130 is positioned in the non-pressing position P1. The retracted position P3 of the force transmission member 140 is an example of a third position in the claims.

As shown in FIG. 5, when the force transmission member 140 slides diagonally upward from the retracted position P3 along the operation direction D2, and moves to an advanced position P4 closer to the through-member 130 than the retracted position P3, the force transmission member 140 maintains a state where the guide protrusion 135 of the through-member 130 is fitted in the guide groove 144 of the force transmission member 140, performs a relative upward sliding movement with respect to the through-member 130, and presses and displaces the through-member 130 toward the distal end side. When the force transmission member 140 is in the advanced position P4, the force transmission member 140 positions the through-member 130 in the pressing position P2 as a result of pressing the through-member 130. The advanced position P4 is an example of a fourth position in the claims.

The holding mechanism 160 (FIG. 8) adopts a so-called double knock mechanism, and switches between a state in which the force transmission member 140 is held in the retracted position P3 and a state in which the force transmission member 140 is held in the advanced position P4 each time the operating member 180 slides along the operation direction D2. The holding mechanism 160 includes an outer cylinder 150, a rotor 170, and a biasing member 162. In the present embodiment, a portion of the operating member 180 constitutes a portion of the holding mechanism 160.

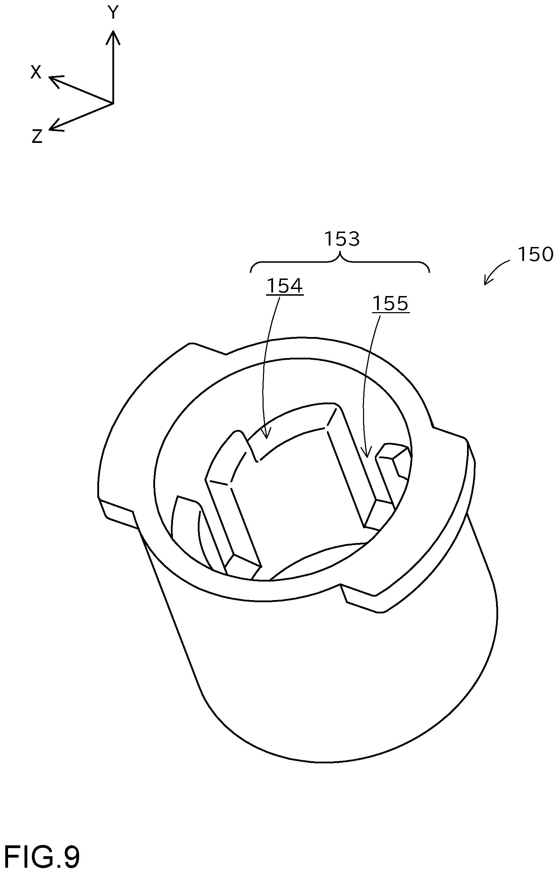

FIG. 9 is a perspective view showing an external configuration of the outer cylinder 150 of the first embodiment. The outer cylinder 150 is a substantially hollow cylindrical member, and for example, is formed of a resin. As shown in FIG. 3, the outer cylinder 150 is attached to the distal end portion of a part of the housing 110 in which the member accommodating space 116 is formed, in a posture in which the axial direction coincides with the operation direction D2. As shown in FIG. 9, the inner peripheral surface of the outer cylinder 150 is formed having a tooth-shaped outer cylinder end surface cam 153. The outer cylinder end surface cam 153 is a cam formed having alternating shallow groove portions 154 and deep groove portions 155 in the circumferential direction. In the present embodiment, the outer cylinder end surface cam 153 is configured by four shallow groove portions 154 and four deep groove portions 155.

A tooth-shaped operating member end surface cam 182 is formed on the end surface on the upper distal end side of the substantially hollow cylindrical operating member 180. A plurality of (four) sliding contactors 181, which are substantially rectangular parallelepiped projections, are formed on the outer peripheral surface of the operating member 180. The operating member 180 is inserted into the hollow portion of the outer cylinder 150, and the sliding contactors 181 on the outer peripheral surface of the operating member 180 are fitted into the deep groove portions 155 on the inner peripheral surface of the outer cylinder 150. As a result, the operating member 180 is capable of sliding along the axial direction of the outer cylinder 150, that is, along the operation direction D2 in a state where rotation is restricted with respect to the outer cylinder 150.

The rotor 170 is a substantially circular plate-shaped member, and for example, is formed of a resin. The outer peripheral surface of the rotor 170 is formed having a plurality of (four) protrusions 173. The surface on the lower proximal end side of the protrusions 173 of the rotor 170 is formed having teeth. Here, the pitch of the peak parts of the operating member end surface cam 182 of the operating member 180 is offset by approximately half with respect to the pitch of the peak parts of the outer cylinder end surface cam 153 of the outer cylinder 150, and has a structure in which the protrusions 173 of the rotor 170 are unable to simultaneously engage with both the operating member end surface cam 182 and the outer cylinder end surface cam 153. Furthermore, the surface on the upper distal end side of the rotor 170 is formed having a recess 174.

The biasing member 162 is a substantially hollow cylindrical spring, and is formed of a metal such as stainless steel. The biasing member 162 is disposed between the rotor 170 and the force transmission member 140 (FIG. 7), and biases the force transmission member 140 along the operation direction D2 toward the external side of the housing 110. One end portion of the biasing member 162 is inserted into the recess 174 of the rotor 170, and the protrusion 142 of the force transmission member 140 is inserted into a hollow portion of another end portion of the biasing member 162.

In a rotor-retracted state, in which the protrusions 173 of the rotor 170 are fitted in the deep groove portions 155 of the outer cylinder end surface cam 153 of the outer cylinder 150, the rotor 170 is positioned in a position that is retracted to the lower proximal end side with the deep groove portions 155 as a guide. Therefore, in the rotor-retracted state, as shown in FIGS. 3 and 4, the force transmission member 140 is positioned in the retracted position P3 that is retracted to the lower proximal end side, and as a result, the through-member 130 is positioned in the non-pressing position P1. The rotor-retracted state is maintained as long as the operating member 180 does not slide.

In the rotor-retracted state, when the operating member 180 advances along the operation direction D2 to the upper distal end side, the operating member end surface cam 182 of the operating member 180 engages the protrusions 173 of the rotor 170, which causes the rotor 170 to also advance with the operating member 180 in the same direction. When the rotor 170 advances to a position in which the protrusions 173 escape from the deep groove portions 155, the rotor 170 rotates in the circumferential direction by one-half of a peak due to the protrusions 173 sliding along the shape of the teeth of the operating member end surface cam 182. As a result, the protrusions 173 and the operating member end surface cam 182 are completely engaged with each other. Then, when the operating member 180 retracts along the operation direction D2 to the lower proximal end side, the rotor 170 also retracts in the same direction with the operating member 180. At this time, because the rotor 170 has already rotated by one-half of a peak, the protrusions 173 are fitted in the shallow groove portions 154 of the outer cylinder end surface cam 153 of the outer cylinder 150 rather than the deep groove portions 155. In a rotor-advanced state in which the protrusions 173 are fitted in the shallow groove portions 154, the rotor 170 is held in a position that is advanced to the upper distal end side compared to the rotor-retracted state in which the protrusions 173 are fitted in the deep groove portions 155. Therefore, in the rotor-advanced state, as shown in FIG. 5, the force transmission member 140 advances to the advanced position P4, and as a result, the through-member 130 moves to the pressing position P2. The rotor-advanced state is maintained as long as the operating member 180 does not slide.

In the rotor-advanced state, when the operating member 180 advances along the operation direction D2 to the upper distal end side, the operating member end surface cam 182 engages the protrusions 173 of the rotor 170, which causes the rotor 170 to also advance with the operating member 180 in the same direction. When the rotor 170 advances to a position in which the protrusions 173 escape from the shallow groove portions 154, the rotor 170 rotates in the circumferential direction by one-half of a peak due to the protrusions 173 sliding along the tooth shape of the operating member end surface cam 182. As a result, the protrusions 173 and the operating member end surface cam 182 are completely engaged with each other. Then, when the operating member 180 retracts along the operation direction D2 to the lower proximal end side, the rotor 170 also retracts in the same direction with the operating member 180. At this time, because the rotor 170 has already rotated by one-half of a peak, the protrusions 173 are fitted in the deep groove portions 155 of the outer cylinder end surface cam 153 rather than the shallow groove portions 154. As a result, the holding mechanism 160 returns to the rotor-retracted state, the force transmission member 140 returns to the retracted position P3, and the through-member 130 moves to the non-pressing position P1. In this way, the state of the holding mechanism 160 switches between a state in which the force transmission member 140 is held in the retracted position P3 and a state in which the force transmission member 140 is held in the advanced position P4 each time the operating member 180 slides along the operation direction D2.

### A-3. Motion of Opening/Closing Mechanism 100:

Next, the motion of the opening/closing mechanism 100 for the hemostasis valve 120 will be described. In the initial state, as shown in FIGS. 3 and 4, the holding mechanism 160 is in the rotor-retracted state. In this state, the force transmission member 140 is positioned in the retracted position P3, and the through-member 130 is not pressed by the force transmission member 140 and is positioned in the non-pressing position P1. Therefore, the hemostasis valve 120 is closed, and the opening/closing mechanism 100 is the closed state opening/closing mechanism 100c.

For example, when the thumb of the operator that is gripping the medical connector 10 applies an operation (hereinafter, referred to as "valve-opening operation") of pressing the operating member 180 along the operation direction D2 to the inner side of the housing 110 (that is, diagonally upward), the operating member 180 slides diagonally upward along the operation direction D2. Accordingly, the holding mechanism 160 switches from the rotor-retracted state to the rotor-advanced state. In this state, as shown in FIG. 5, the force transmission member 140 is positioned in the advanced position P4, and the through-member 130 is pressed by the force transmission member 140 and is positioned in the pressing position P2. As a result, the distal end portion of the main body portion 131 of the through-member 130 presses the hemostasis valve 120, which places the hemostasis valve 120 in the open state, and the opening/closing mechanism 100 becomes the open state opening/closing mechanism 100o.

Furthermore, when the opening/closing mechanism 100 is the open state opening/closing mechanism 100o, for example, when the thumb of the operator applies, in the same manner as the valve-opening operation, an operation (hereinafter, referred to as "valve-closing operation") of pressing the operating member 180 along the operation direction D2 to the inner side of the housing 110 (that is, diagonally upward), the operating member 180 slides diagonally upward along the operation direction D2. Accordingly, the holding mechanism 160 switches from the rotor-advanced state to the rotor-retracted state. In this state, as shown in FIGS. 3 and 4, the force transmission member 140 is positioned in the retracted position P3, and the through-member 130 is not pressed by the force transmission member 140 and returns to the non-pressing position P1. As a result, the through-member 130 does not press the hemostasis valve 120, which causes the hemostasis valve 120 to be placed in the closed state, and the opening/closing mechanism 100 becomes the closed state opening/closing mechanism 100c.

In this way, the state of the opening/closing mechanism 100 switches to the closed state opening/closing mechanism 100c or the open state opening/closing mechanism 100o each time the pressing operation (the valve-opening operation and the valve-closing operation) is performed with respect to the operating member 180.

### A-4. Technical Effects of Opening/Closing Mechanism

As described above, in the opening/closing mechanism 100, the holding mechanism 160 switches between a state in which the force transmission member 140 is held in the retracted position P3 and a state in which the force transmission member 140 is held in the advanced position P4 each time the operating member 180 slides along the operation direction D2. When the holding mechanism 160 switches to a state in which the force transmission member 140 is held in the advanced position P4, the through-member 130 moves from the non-pressing position P1 to the pressing position P2, and the hemostasis valve 120 switches from the closed state to the open state. At this time, because the holding mechanism 160 holds the force transmission member 140 in the advanced position P4, the open state of the hemostasis valve 120 is maintained. Furthermore, when the holding mechanism 160 switches with the sliding of the operating member 180 once again to a state in which the force transmission member 140 is held in the retracted position P3, the through-member 130 moves from the pressing position P2 to the non-pressing position P1, and the hemostasis valve 120 switches from the open state to the closed state. At this time, because the holding mechanism 160 holds the force transmission member 140 in the retracted position P3, the closed state of the hemostasis valve 120 is maintained. In the opening/closing mechanism 100, because the operating member 180 is capable of sliding along the operation direction D2, which is not parallel to the extending direction (Z-axis direction) of the lumen 113 of the housing 110, the opening and closing operation of the hemostasis valve 120 becomes an operation of pressing the operating member 180 in the operation direction D2, which is not parallel to the extending direction of the lumen 113. As a result, according to the opening/closing mechanism 100, it is possible for an operator to easily perform the opening and closing operation of the hemostasis valve 120 with the thumb while gripping the medical connector 10, and the operability of the opening/closing mechanism 100 can be improved.

The holding mechanism 160 includes the cylindrical biasing member 162 that biases the force transmission member 140 along the operation direction D2 toward the external side of the housing 110, and the surface of the force transmission member 140 facing the biasing member 162 is formed having the protrusion 142 that is inserted into a hollow portion of the biasing member 162. Therefore, according to the opening/closing mechanism 100, it is possible to easily and accurately perform the positioning between the force transmission member 140 and the biasing member 162, and it is also possible to effectively transmit the biasing force of the biasing member 162 to the force transmission member 140, which enables the accuracy of the motion of the opening/closing mechanism 100 to be improved.

The angle formed between the extending direction (Z-axis direction) of the lumen 113 of the housing 110 and the operation direction D2 of the operating member 180 is 35 degrees or more and 55 degrees or less. As a result, according to the opening/closing mechanism 100, it is possible for an operator to very easily operate the operating member 180 with the thumb while gripping the medical connector 10, and the operability of the opening/closing mechanism 100 can be effectively improved.

The through-member 130 includes the main body portion 131 in which the through-hole 132 is formed, and the flange portion 133 that protrudes in the up-down direction from the main body portion 131, and the force transmission member 140 makes contact with the surface on the proximal end side of the flange portion 133 of the through-member 130 and is configured to be capable of a relative sliding movement with respect to the through-member 130 along the up-down direction. Therefore, according to the opening/closing mechanism 100, the sliding of the force transmission member 140 along the operation direction D2 not parallel to the extending direction (Z-axis direction) of the lumen 113 of the housing 110 and the sliding of the through-member 130 in the extending direction (Z-axis direction) can be efficiently converted, and the operability of the opening/closing mechanism 100 can be effectively improved.

### A-5. Modification of Opening/Closing Mechanism

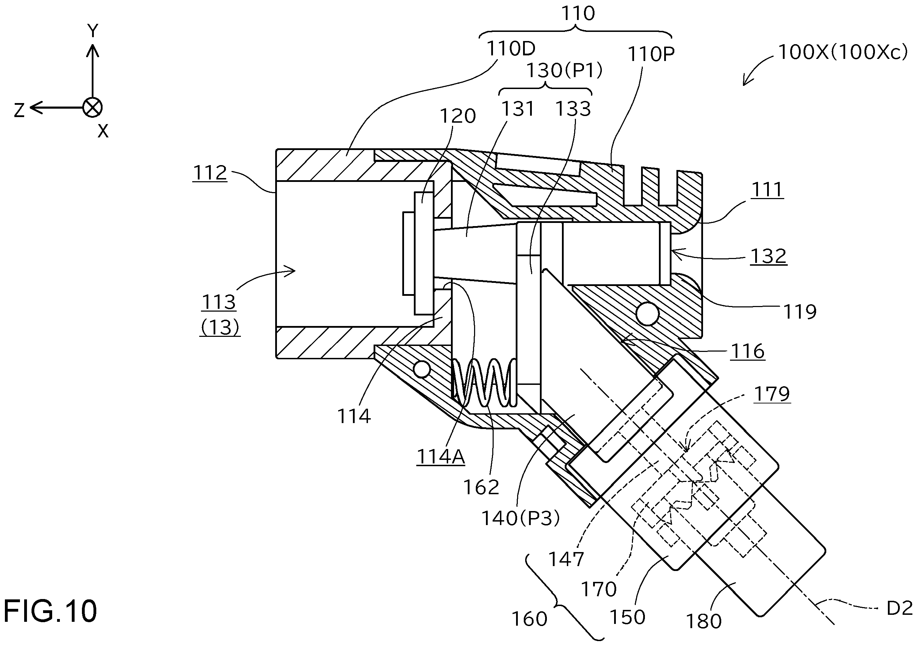

FIG. 10 is an explanatory diagram showing a configuration of a modification (opening/closing mechanism 100X) of the opening/closing mechanism 100 of the first embodiment. The opening/closing mechanism 100X differs from the opening/closing mechanism 100 in that the operating member 180 and the force transmission member 140 are an integrated member. Specifically, a connection shaft 147 extends from the surface on the lower proximal end side of the force transmission member 140 to the lower proximal end side, and the connection shaft 147 passes through the through-hole 179 formed in the rotor 170 up to the operating member 180, and is connected to the operating member 180. As a result, in the opening/closing mechanism 100X, the operating member 180 and the force transmission member 140 are integrated and slide along the operation direction D2. Furthermore, in the opening/closing mechanism 100X, the biasing member 162 is disposed substantially parallel to the front-rear direction between the distal end side surface on the lower portion of the flange portion 133 of the through-member 130 and the housing 110. As a result of the biasing member 162 biasing the through-member 130 to the proximal end side, it indirectly biases the force transmission member 140 along the operation direction D2 toward the external side of the housing 110. In the opening/closing mechanism 100X according to such a modification, the holding mechanism 160 switches between a state in which the force transmission member 140 is held in the retracted position P3 and a state in which the force transmission member 140 is held in the advanced position P4 each time the operating member 180 slides along the operation direction D2. Therefore, according to the opening/closing mechanism 100X, the operability of the opening/closing mechanism 100X can be similarly improved.

### A-6. Configuration of Fixing Mechanism for Long Medical Device:

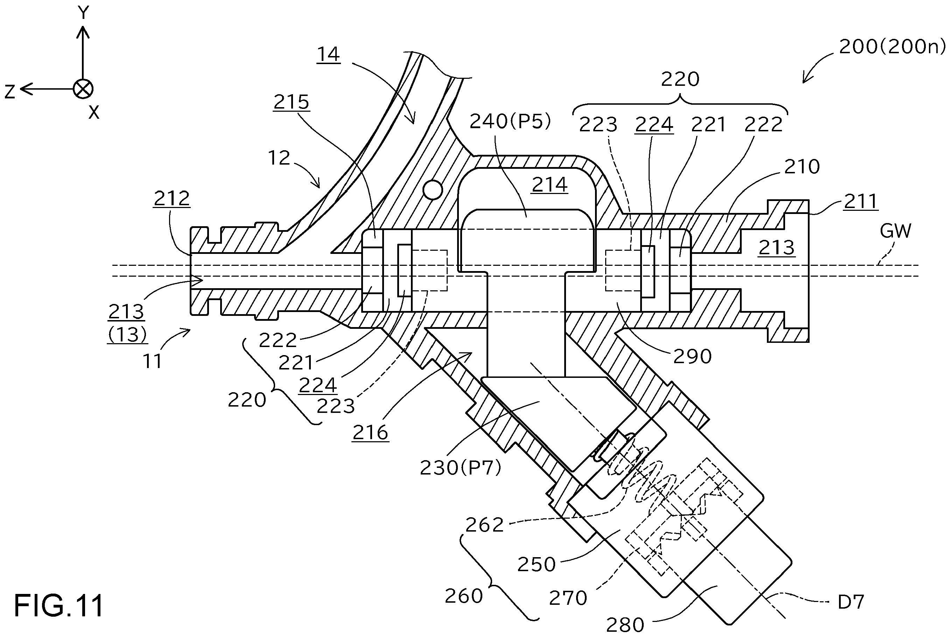

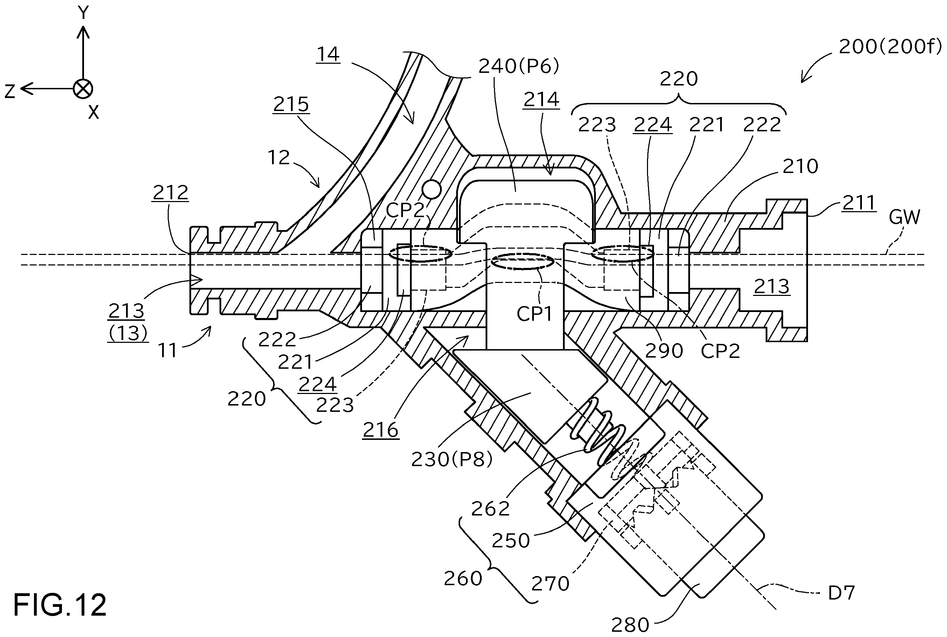

Next, the configuration of the fixing mechanism 200 for a long medical device will be described. FIGS. 11 and 12 are explanatory diagrams showing the configuration. FIGS. 11 and 12 show the configuration of a longitudinal cross-section (YZ cross-section). FIG. 11 shows the fixing mechanism 200 in a state in which the fixing of a long medical device such as the guide wire GW has been released (hereinafter referred to as "released state fixing mechanism 200n"), and FIG. 12 shows the fixing mechanism 200 in a state in which a long medical device has been fixed (hereinafter referred to as "fixed state fixing mechanism 200f').

The fixing mechanism 200 is a mechanism for fixing, and releasing the fixing of a long medical device inserted through the lumen 13 of the main pipe portion 11 (FIG. 2) of the medical connector 10. The fixing mechanism 200 is a mechanism that switches between a fixed state that fixes the long medical device and an unfixed state in which the fixing of the long medical device is released each time a pressing operation is performed by an operator with respect to the operating member 280. The fixing mechanism 200 includes a housing 210, a cylindrical body 290, a pressing member 240, an operating member 280, a force transmission member 230, and a holding mechanism 260.

The housing 210 is formed having a distal end side opening 212 and a proximal end side opening 211, and is a tubular member formed having a lumen 213 that communicates with the distal end side opening 212 and the proximal end side opening 211. The lumen 213 is a through-hole extending in the front-rear direction (Z-axis direction), and constitutes a portion of the lumen 13 of the medical connector 10. The housing 210, for example, is formed of a resin. A branched pipe portion 12 is formed on a distal end portion of the housing 210. Furthermore, as shown in FIG. 2, a proximal end portion of the housing 210 is inserted and fixed to the lumen 113 at the distal end portion of the housing 110 of the opening/closing mechanism 100. The movement toward the distal end side of the hemostasis valve 120 is regulated by the proximal end portion of the housing 210.

A cylindrical body accommodating space 215, which is a part of the lumen 213 having an increased diameter, is formed inside the housing 210 in the vicinity of the center in the front-rear direction. Furthermore, a retraction space 214 that communicates with the vicinity of the center in the front-rear direction is formed above the cylindrical body accommodating space 215. Furthermore, a member accommodating space 216 is formed below the cylindrical body accommodating space 215, which communicates with the vicinity of the center in the front-rear direction, and extends in a diagonal direction toward the lower proximal end side and is open at the surface of the housing 210.

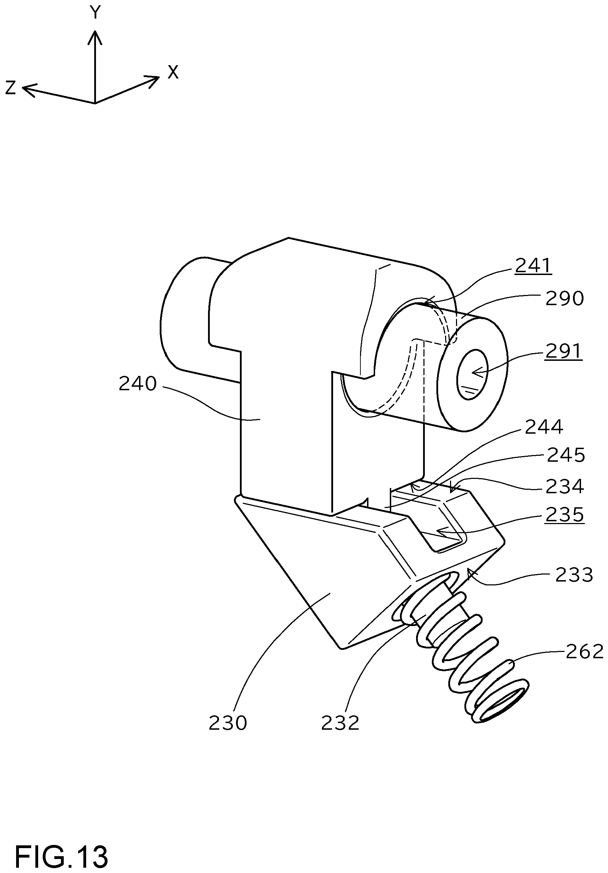

FIG. 13 is a cross-sectional perspective view showing a configuration of the cylindrical body 290, the pressing member 240, and the force transmission member 230 of the first embodiment. The cylindrical body 290 is a flexible cylindrical member formed having a through-hole 291 into which a long medical device such as the guide wire GW is inserted. The cylindrical body 290 is formed, for example, of an elastic material such as silicone rubber. As shown in FIGS. 11 and 12, the cylindrical body 290 is attached inside the cylindrical body accommodating space 215 with a posture in which the axis is parallel to the front-rear direction (Z-axis direction). In the cylindrical body 290, the through-hole 291 communicates with the distal end side opening 212 and the proximal end side opening 211 of the housing 210. Furthermore, the through-hole 291 and the lumen 213 of the housing 210 are coaxial with each other. The front-rear direction (Z-axis) direction is an example of a fifth direction in the claims.

Substantially cylindrical stoppers 220 are attached to both ends of the cylindrical body 290. Each stopper 220 includes a main body portion 221 having substantially the same diameter as the cylindrical body 290, a first small diameter portion 223 that is positioned on the cylindrical body 290 side of the main body portion 221 and has a smaller diameter than the main body portion 221, and a second small diameter portion 222 that is positioned on the opposite side of the main body portion 221 to the cylindrical body 290 and has a smaller diameter than the main body portion 221. The first small diameter portion 223 of each stopper 220 is inserted into the through-hole 291 of the cylindrical body 290. Furthermore, the main body portion 221 of each stopper 220 is formed having a recess 224 to which a protrusion (not illustrated) of the housing 210 is fitted. As a result of the fitting between the protrusion and the recess 224, each stopper 220 is positioned in the front-rear direction with respect to the housing 210, and consequently, the cylindrical body 290 is positioned in the front-rear direction with respect to the housing 210.

The pressing member 240 is a member formed having a through-hole 241 extending in the front-rear direction, and for example, is formed of a resin. The pressing member 240 has a substantially rectangular parallelepiped shape, and the length (dimension in the front-rear direction) of the part on the upper side of the center position of the through-hole 241 is longer than that of the other part. The inner diameter of the through-hole 241 of the pressing member 240 is substantially the same as the outer diameter of the cylindrical body 290, and the cylindrical body 290 is inserted into the through-hole 241. Furthermore, a lower surface 244 of the pressing member 240 is a substantially flat surface that is substantially orthogonal to the up-down direction, and is formed having a guide protrusion 245 continuously extending in the front-rear direction.

The pressing member 240 is accommodated across the retraction space 214, the cylindrical body accommodating space 215, and the member accommodating space 216 of the housing 210. The pressing member 240 is capable of sliding along the up-down direction (Y-axis direction), which is a direction orthogonal to the axial direction (Z-axis direction) of the cylindrical body 290. The pressing member 240, which is capable of sliding in the up-down direction, can be positioned in a non-pressing position P5 shown in FIG. 11, in which the through-hole 241 of the pressing member 240 becomes coaxial with the through-hole 291 of the cylindrical body 290, and a pressing position P6 shown in FIG. 12, which is upwardly displaced form the non-pressing position P5. When the pressing member 240 is positioned in the non-pressing position P5 (FIG. 11), the pressing member 240 substantially does not press the cylindrical body 290. Here, the state of substantially not pressing includes, in addition to a state where the inner peripheral surface of the through-hole 241 of the pressing member 240 and the outer peripheral surface of the cylindrical body 290 are separated, a state where the inner peripheral surface of the through-hole 241 of the pressing member 240 and the outer peripheral surface of the cylindrical body 290 are in close contact because the inner diameter of the through-hole 241 of the pressing member 240 is the same as, or slightly smaller than, the outer shape of the cylindrical body 290 before insertion into the through-hole 241. The up-down direction (Y-axis direction) is an example of a sixth direction in the claims, and the non-pressing position P5 is an example of a fifth position in the claims.

On the other hand, when the pressing member 240 is positioned in the pressing position P6 (FIG. 12), the pressing member 240 upwardly presses a part of the cylindrical body 290 excluding both end portions (a center part in the front-rear direction in the present embodiment) from the outer peripheral side, and causes the cylindrical body 290 to undergo elastic deformation. In this state, the inner peripheral surface of the elastically deformed cylindrical body 290 (primarily a contact position CP1, being a lower side part on the inner peripheral surface of the deformed part of the cylindrical body 290) is pressed against the long medical device, and because the long medical device is supported by shearing at a total of three positions, namely the contact position CP1, and contact portions CP2, being the upper part of the inner peripheral surface of both end portions of the cylindrical body 290 (or the stoppers 220), the long medical device is fixed by the fixing mechanism 200 and sliding is regulated in the front-rear direction. The retraction space 214 of the housing 210 has a sufficient area for the pressing member 240 to deform the cylindrical body 290 to an extent that allows the long medical device to be fixed. The pressing position P6 is an example of a sixth position in the claims.

As shown in FIG. 11, the operating member 280 is a substantially hollow cylindrical member extending in a direction (hereinafter referred to as "operation direction D7") not parallel to the front-rear direction (Z-axis direction). In the present embodiment, the operation direction D7 is a direction at an angle of 35 degrees or more and 55 degrees or less with respect to the front-rear direction, and for example, is a direction at an angle of 45 degrees with respect to the front-rear direction (a direction inclined at 45 degrees from the upper distal end side toward the lower proximal end side). The operating member 280 is supported by an outer cylinder 250 of the holding mechanism 260 described below, and is capable of sliding along the operation direction D7. A part of the operating member 280 on the proximal end side is exposed from the outer cylinder 250, and enables a pressing operation to be made by an operator such as a physician. The operating member 280, for example, is formed of a resin. The operation direction D7 is an example of a seventh direction in the claims.

As shown in FIG. 13, the force transmission member 230 is a substantially polygonal member, and for example, is formed of a resin. An upper surface 234 of the force transmission member 230 is a substantially flat surface that is substantially orthogonal to the up-down direction, and is formed having a guide groove 235 continuously extending in the front-rear direction. The guide protrusion 245 of the pressing member 240 is fitted into the guide groove 235. Furthermore, a surface 233 on the lower proximal end side of the force transmission member 230 is formed having a projection-shaped protrusion 232.

As shown in FIGS. 11 and 12, the force transmission member 230 is accommodated so as to be positioned between the pressing member 240 and the operating member 280 in the member accommodating space 216 of the housing 210, and is capable of sliding along the operation direction D7. The force transmission member 230 is capable of transmitting to the pressing member 240 a force along the operation direction D7 that causes the operating member 280 to approach the pressing member 240.

Because the force transmission member 230 has the configuration described above, it makes contact with the lower surface 244 of the pressing member 240, while also being configured to be capable of a relative sliding movement with respect to the pressing member 240 along the front-rear direction (Z-axis direction). That is, as shown in FIG. 11, when the pressing member 240 is in the non-pressing position P5 where it does not press the cylindrical body 290, the force transmission member 230 is in a retracted position P7 where it is retracted to the lower proximal end side. When the force transmission member 230 is in the retracted position P7, the force transmission member 230 does not press the pressing member 240. When the pressing member 240 is not being pressed by the force transmission member 230, the pressing member 240 is positioned in the non-pressing position P5 due to an elastic restoring force of the cylindrical body 290. The retracted position P7 is an example of a seventh position in the claims.

Furthermore, as shown in FIG. 12, when the force transmission member 230 slides diagonally upward from the retracted position P7 along the operation direction D7, and moves to an advanced position P8 closer to the cylindrical body 290 than the retracted position P7, the force transmission member 230 maintains a state where the guide protrusion 245 of the pressing member 240 is fitted in the guide groove 235 of the force transmission member 230, and performs a relative sliding movement with respect to the pressing member 240 toward the distal end side. When the force transmission member 230 is in the advanced position P8, the force transmission member 230 places the pressing member 240 in the pressing position P6 as a result of pressing the pressing member 240. The advanced position P8 is an example of an eighth position in the claims.

The holding mechanism 260 adopts a so-called double knock mechanism, and switches between a state in which the force transmission member 230 is held in the retracted position P7 and a state in which the force transmission member 230 is held in the advanced position P8 each time the operating member 280 slides along the operation direction D7. The holding mechanism 260 includes an outer cylinder 250, a rotor 270, and a biasing member 262. In the present embodiment, a portion of the operating member 280 constitutes a portion of the holding mechanism 260. Because the configuration of the holding mechanism 260 is the same as the configuration of the holding mechanism 160 of the opening/closing mechanism 100 described above, the description will be omitted. That is, in the description of the configuration of the holding mechanism 160 of the opening/closing mechanism 100 above, the outer cylinder 150 may be interpreted as being the outer cylinder 250, the rotor 170 may be interpreted as being the rotor 270, and the biasing member 162 may be interpreted as being the biasing member 262.

### A-7. Motion of Fixing Mechanism:

Next, the motion of the fixing mechanism 200 for a long medical device will be described. In the initial state, as shown in FIG. 11, the holding mechanism 260 is in the rotor-retracted state. In this state, the force transmission member 230 is positioned in the retracted position P7, and the pressing member 240 is not pressed by the force transmission member 230 and is positioned in the non-pressing position P5. Furthermore, because the cylindrical body 290 is not elastically deformed, a long medical device such as the guide wire GW that has been inserted into the lumen 213 of the housing 210 (the lumen 13 of the main pipe portion 11) is not fixed. That is, the fixing mechanism 200 is the released state fixing mechanism 200n.

For example, when the thumb of the operator that is gripping the medical connector 10 applies an operation (hereinafter, referred to as "fixing operation") of pressing the operating member 280 diagonally upward along the operation direction D7, the operating member 280 slides diagonally upward along the operation direction D7. Accordingly, the holding mechanism 260 switches from the rotor-retracted state to the rotor-advanced state. In this state, as shown in FIG. 12, the force transmission member 230 is positioned in the advanced position P8, and the pressing member 240 is pressed by the force transmission member 230 and is positioned in the pressing position P6. Moreover, the cylindrical body 290 is pressed by the pressing member 240 and undergoes elastic deformation, and the long medical device that has been inserted into the lumen 213 of the housing 210 is fixed. As a result, the fixing mechanism 200 becomes the fixed state fixing mechanism 200f.

Furthermore, when the fixing mechanism 200 is the fixed state fixing mechanism 200f, for example, when the thumb of the operator applies, in the same manner as the fixing operation, an operation (hereinafter, referred to as "unfixing operation") of pressing the operating member 280 along the operation direction D7 diagonally upward, the operating member 280 slides diagonally upward along the operation direction D7. Accordingly, the holding mechanism 260 switches from the rotor-advanced state to the rotor-retracted state. In this state, as shown in FIG. 11, the force transmission member 230 is positioned in the retracted position P7, and the pressing member 240 is not pressed by the force transmission member 230 and returns to the non-pressing position P5 due to the elastic restoring force of the cylindrical body 290. Moreover, the cylindrical body 290 is not pressed by the pressing member 240 and does not undergo elastic deformation, and the fixing of the long medical device that has been inserted into the lumen 213 of the housing 210 is released. As a result, the fixing mechanism 200 returns to the released state fixing mechanism 200n.

In this way, the fixing mechanism 200 switches to the released state fixing mechanism 200n or the fixed state fixing mechanism 200f each time the pressing operation (the fixing operation and the unfixing operation) is performed with respect to the operating member 280.

### A-8. Technical Effects of Fixing Mechanism:

As described above, in the fixing mechanism 200 of the present embodiment, because the operating member 280 is capable of sliding along the operation direction D7, which is not parallel to a direction parallel to the axis of the cylindrical body 290 (Z-axis direction), the fixing operation and unfixing operation of the long medical device becomes an operation of pressing the operating member 280 in the operation direction D7, which is not parallel to a direction parallel to the axis of the cylindrical body 290. Therefore, according to the fixing mechanism 200 of the present embodiment, it is possible to achieve fixing and unfixing of the long medical device with a simple operation in which the operator presses the operating member 280 with the thumb while gripping the medical connector 10, and because the degree to which the long medical device is fixed can be grasped visually or by feel, the operability of the fixing mechanism 200 can be improved.

The holding mechanism 260 includes the cylindrical biasing member 262 that biases the force transmission member 230 in a direction that approaches the pressing member 240, and the surface facing the biasing member 262 is formed having a protrusion 232 that is inserted into the hollow portion of the biasing member 262. According to fixing mechanism 200 of the present embodiment, it is possible to easily and accurately perform the positioning between the force transmission member 230 and the biasing member 262, and it is also possible to effectively transmit the biasing force of the biasing member 262 to the force transmission member 230, which enables the accuracy of the motion of the fixing mechanism 200 to be improved.

The angle formed between the direction parallel to the axis of the cylindrical body 290 (Z-axis direction) and the operation direction D7 of the operating member 280 is 35 degrees or more and 55 degrees or less. As a result, according to the fixing mechanism 200 of the present embodiment, it is possible for an operator to very easily operate the operating member 280 with the thumb while gripping the medical connector 10, and the operability of the fixing mechanism 200 can be effectively improved.

The force transmission member 230 makes contact with the surface of the pressing member 240, while also being configured to be capable of a relative sliding movement with respect to the pressing member 240 along a direction (Z-axis direction) parallel to the axis of the cylindrical body 290. Therefore, according to the fixing mechanism 200 of the present embodiment, the sliding of the force transmission member 230 along the operation direction D7 and the sliding of the pressing member 240 along the up-down direction can be efficiently converted, and the operability of the fixing mechanism 200 can be effectively improved.

### B. Second embodiment:

### B-1. Configuration of Medical Connector:

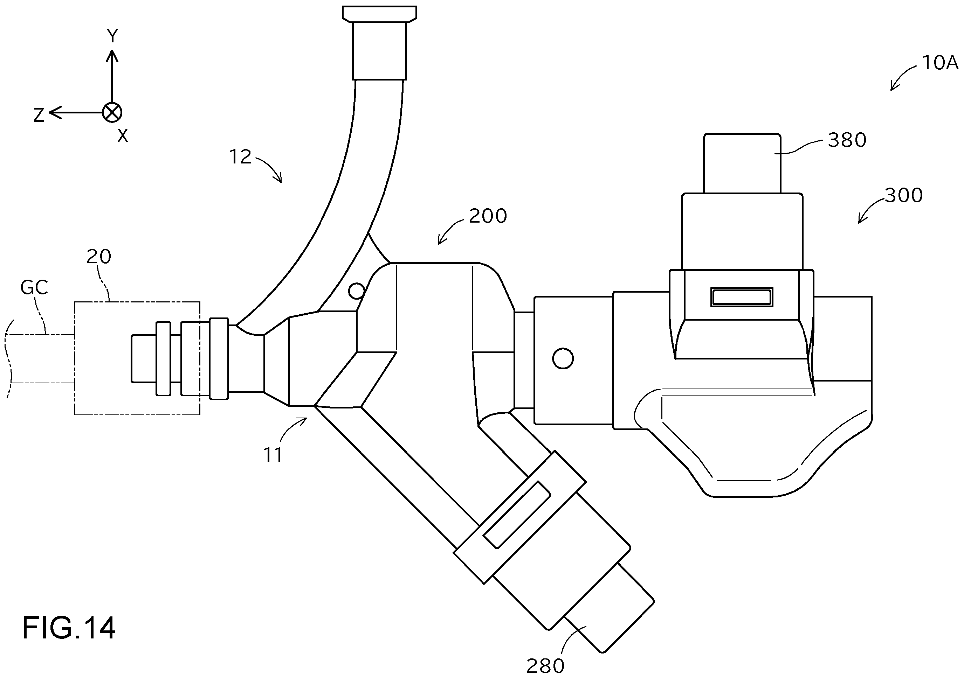

FIG. 14 is an explanatory diagram showing an external configuration of a medical connector 10A in a second embodiment. Hereinafter, those components of the medical connector 10A of the second embodiment that are the same components as those of the medical connector 10 of the first embodiment described above are denoted by the same reference numerals, and the description thereof is omitted as appropriate.

The medical connector 10A includes an opening/closing mechanism 300 and a fixing mechanism 200. The configuration of the fixing mechanism 200 included in the medical connector 10A is the same as the configuration of the fixing mechanism 200 included in the medical connector 10 of the first embodiment. Therefore, the opening/closing mechanism 300 included in the medical connector 10A of the second embodiment will be described below.

### B-2. Configuration of Opening/Closing Mechanism:

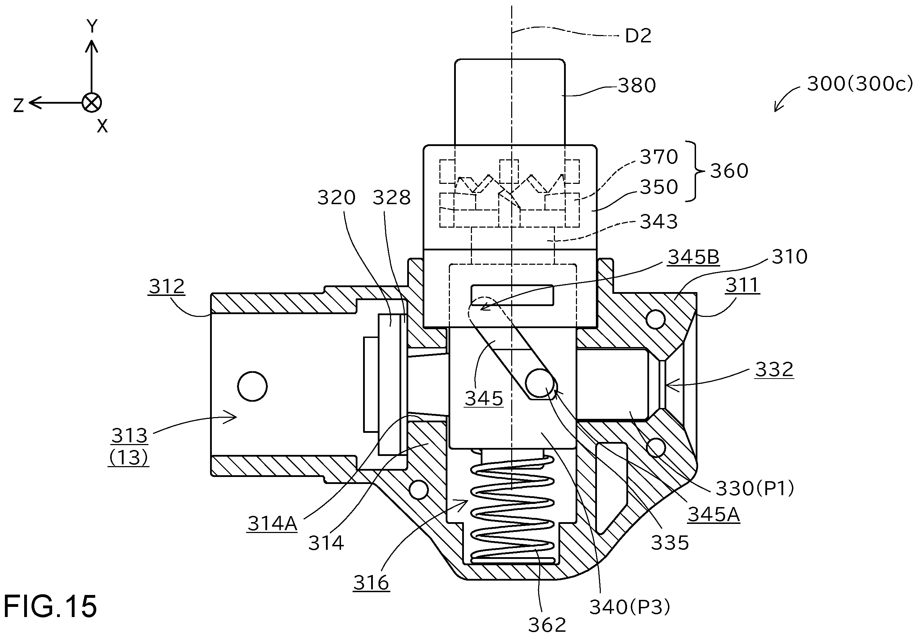

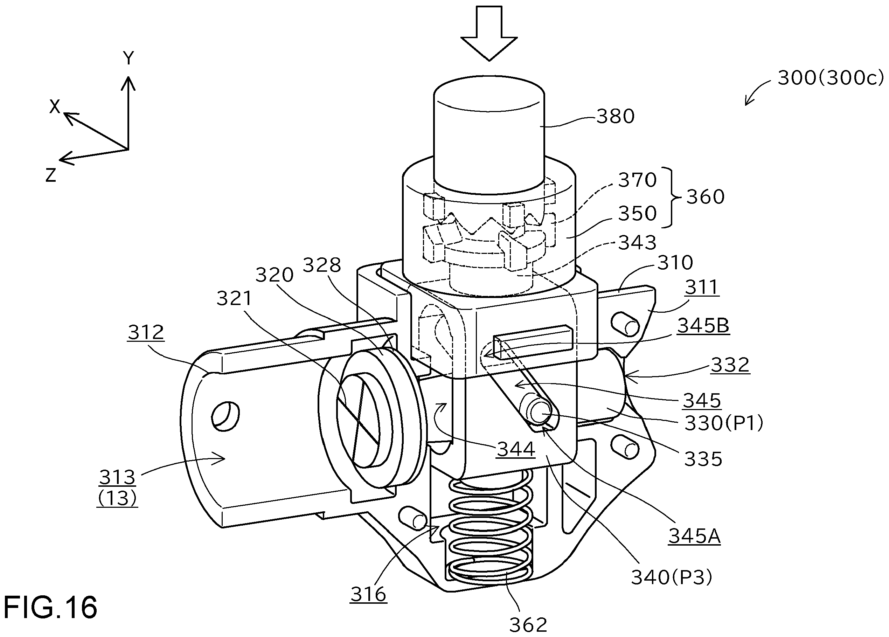

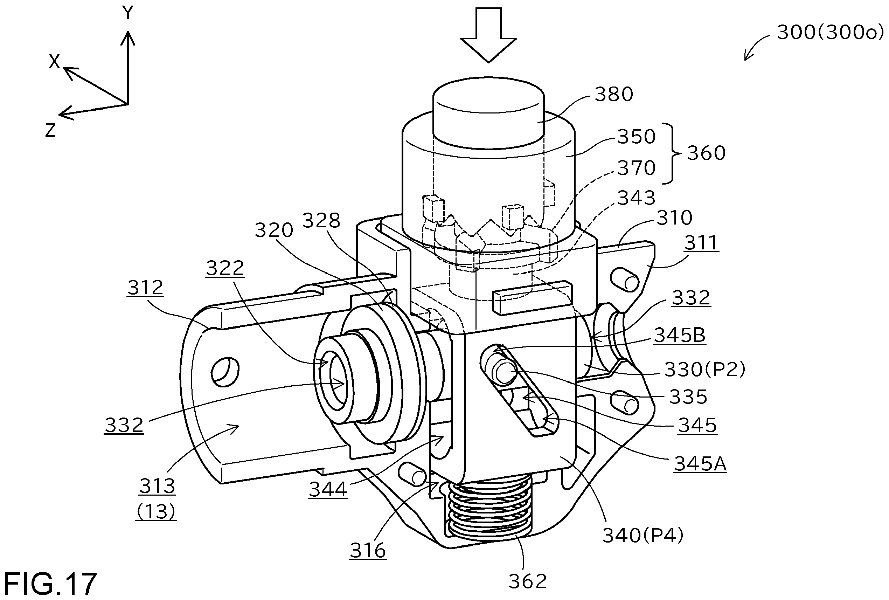

FIGS. 15 to 17 are explanatory diagrams showing the configuration of the opening/closing mechanism 300. FIG. 15 shows the configuration in a longitudinal cross-section (YZ cross-section) of the opening/closing mechanism 300, and FIGS. 16 and 17 show a cross-sectional perspective view of the opening/closing mechanism 300. FIGS. 15 and 16 show the opening/closing mechanism 300 in a state in which the hemostasis valve 320 is closed (hereinafter referred to as "closed state opening/closing mechanism 300c"), and FIG. 17 shows the opening/closing mechanism 300 in a state in which the hemostasis valve 320 is open (hereinafter referred to as "open state opening/closing mechanism 300o).

The opening/closing mechanism 300 is a mechanism for opening and closing the hemostasis valve 320, which suppresses the outflow of blood via the lumen 13 of the main pipe portion 11 (FIG. 14) of the medical connector 10A. The opening/closing mechanism 300 of the present embodiment is a mechanism that switches between a state in which the hemostasis valve 320 is closed or a state in which the hemostasis valve 320 is open state each time the operator performs a pressing operation with respect to the operating member 380. The opening/closing mechanism 300 includes a housing 310, a hemostasis valve 320, a through-member 330, an operating member 380, a force transmission member 340, and a holding mechanism 360.

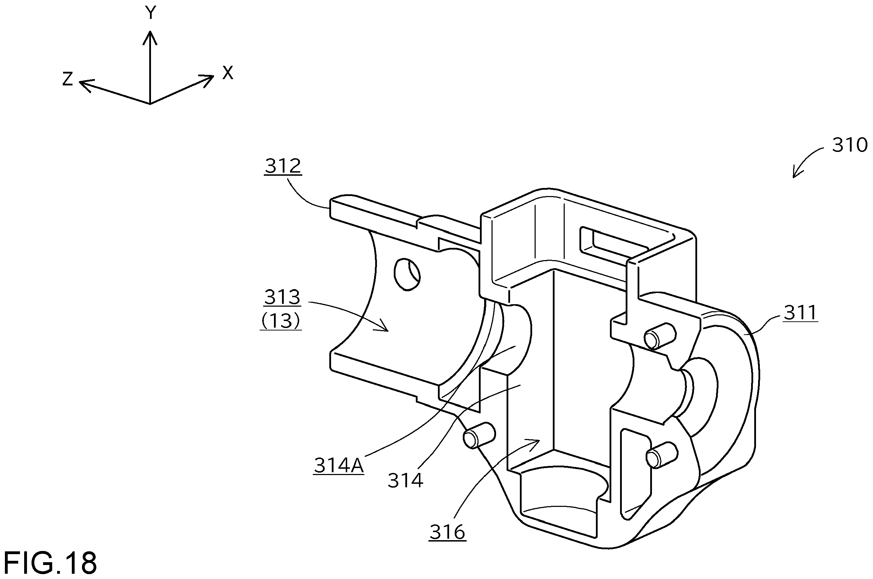

FIG. 18 is a cross-sectional perspective view of a configuration of the housing 310 in the second embodiment. The housing 310 is formed having a distal end side opening 312 and a proximal end side opening 311, and is a tubular member formed having a lumen 313 that communicates with the distal end side opening 312 and the proximal end side opening 311. The lumen 313 formed in the housing 310 is a through-hole extending in the front-rear direction (Z-axis direction), and constitutes a portion of the lumen 13 of the main pipe portion 11 of the medical connector 10A. The housing 310, for example, is formed of a resin. The Z-axis direction is an example of a first direction in the claims.

In the vicinity of the center of the inside of the housing 310 in the front-rear direction, a substantially flat plate-shaped partition wall 314 that is substantially orthogonal to the front-rear direction is formed. The partition wall 314 is formed having a through-hole 314A that passes through the partition wall 314 in the front-rear direction and constitutes a portion of the lumen 313. The transverse cross-sectional shape of the through-hole 314A, for example, is substantially circular. Furthermore, a member accommodating space 316 extending in the up-down direction is formed on the proximal end side of the partition wall 314 inside the housing 310. The member accommodating space 316 is open at the surface of the housing 110 at the upper portion of the housing 110.

The hemostasis valve 320 is a member having the same configuration as the hemostasis valve 120 of the first embodiment. The hemostasis valve 320 is fixed at a position further toward the distal end side than the partition wall 314 inside the housing 310. The hemostasis valve 320 is normally in the closed state in which the slit 321 is closed and the valve is closed (FIGS. 15 and 16). Furthermore, when the hemostasis valve 320 is pressed from the proximal end side, the hemostasis valve 320 switches to the open state, in which a through-hole 322 is formed that passes through the hemostasis valve 320 in front-rear direction (FIG. 17). When the pressing force from the proximal end side is no longer applied, the hemostasis valve 320 elastically deformed and returns to the closed state. In the present embodiment, a substantially annular boss 328 is disposed between the hemostasis valve 320 and the partition wall 314 of the housing 310.

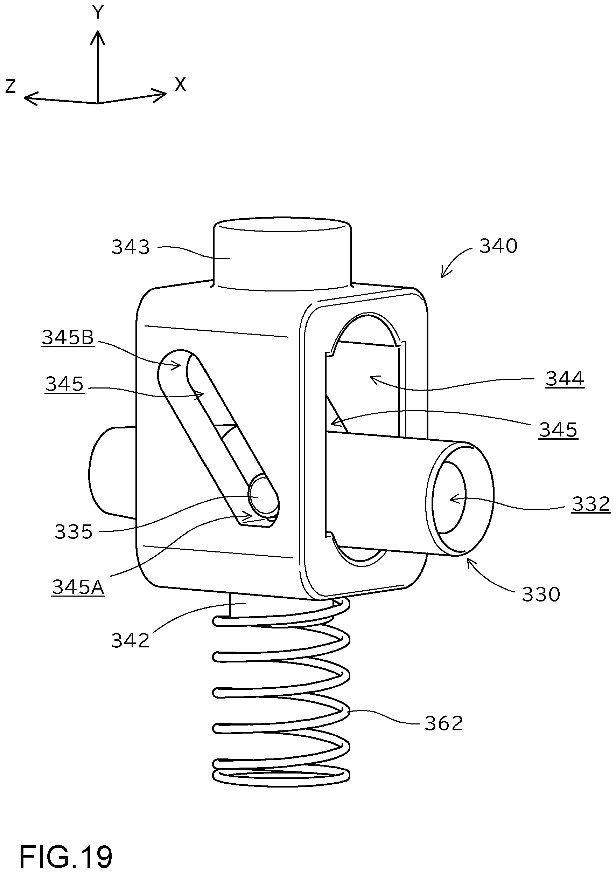

FIG. 19 is a perspective view showing an external configuration of the through-member 330 and the force transmission member 340 of the second embodiment. As shown in FIG. 19, the through-member 330 is a tubular member formed having a through-hole 332 extending in the front-rear direction, and for example, is formed of a resin. The outer peripheral surface of the through-member 330 is formed having protrusions 335 that protrude in the left-right direction (X-axis direction), which is orthogonal to both the front-rear direction and the up-down direction. A total of two protrusions 335 are provided, one on each surface on both sides of the through-member 330. In the present embodiment, the positions of the two protrusions 335 are the same when viewed in the X-axis direction. The left-right direction is an example of a fourth direction in the claims.

As shown in FIG. 15, the through-member 330 is accommodated inside the housing 310 further toward the proximal end side than the hemostasis valve 320. In the through-member 330 accommodated in the housing 310, the through-hole 332 communicates with the proximal end side opening 311 of the housing 310. Furthermore, the through-hole 332 of the through-member 330 and the lumen 313 of the housing 310 are coaxial with each other.

The through-member 330 accommodated in the housing 310 is capable of sliding in the front-rear direction in a state in which the position in the up-down direction and the left-right direction with respect to the housing 310 is fixed. The through-member 330 is positioned such that it faces the through-hole 314A of the partition wall 314 in the front-rear direction. The through-member 330, which is capable of sliding in the front-rear direction, has the distal end portion inserted into the through-hole 314A, and can be positioned in a pressing position P2 that presses the hemostasis valve 320 and places the hemostasis valve 320 in the open position (the state shown in FIG. 17), and a non-pressing position P1 that does not press the hemostasis valve 320 and places the hemostasis valve 320 in the closed position (the state shown in FIGS. 15 and 16). As shown in FIG. 17, in the state in which the through-member 330 is positioned in the pressing position P2, the through-hole 322 formed in the hemostasis valve 320 and the through-hole 332 of the through-member 330 communicate with each other. The pressing position P2 of the through-member 330 is an example of a second position in the claims, and the non-pressing position P1 of the through-member 330 is an example of a first position in the claims.

As shown in FIGS. 15 to 17, the operating member 380 is a substantially hollow cylindrical member extending in a direction (hereinafter referred to as "operation direction D2") not parallel to the front-rear direction (Z-axis direction). The operating member 380 is supported by an outer cylinder 350 of the holding mechanism 360 described below, and is capable of sliding along the operation direction D2. In the present embodiment, the operation direction D2 is an up-down direction that forms an angle of substantially 90 degrees with the front-rear direction. Herein, substantially 90 degrees refers to a range of 90 degrees plus or minus about 5 degrees. A part of the operating member 380 on the proximal end side is exposed from the outer cylinder 350, and enables a pressing operation to be made by an operator such as a physician. The operating member 380, for example, is formed of a resin. The operation direction D2 is an example of a second direction in the claims.

As shown in FIGS. 15 to 17, and in FIG. 19, the force transmission member 340 is a substantially rectangular parallelepiped member, and for example, is formed of a resin. The force transmission member 340 is formed having a through-hole 344 extending in the front-rear direction, and the through-member 330 is inserted into the through-hole 344. Each of the side surfaces on the left and right of the force transmission member 340 is formed having a communication groove 345 that communicates the outer peripheral surface of the force transmission member 340 and the through-hole 344. The communication grooves 345 are orthogonal to the left-right direction, and extend in a direction that is not parallel to both the front-rear direction and the up-down direction. More specifically, the communication grooves 345 extend in a direction from the lower proximal end side toward the upper distal end side. The inclination angle of the communication grooves 345 with respect to the front-rear direction is, for example, 45 degrees. The protrusions 335 of the through-member 330 are slidably inserted into the communication grooves 345. As a result, the through-member 330 is relatively slidable with respect to the force transmission member 340 along the extending direction of the communication grooves 345.

The upper surface of the force transmission member 340 is formed having a protrusion 343 in the form of a projection that upwardly protrudes. Furthermore, the lower surface of the force transmission member 340 is formed having a protrusion 342 in the form of a projection that downwardly protrudes.

As shown in FIG. 15, the force transmission member 340 is accommodated inside the member accommodating space 316 of the housing 310 so as to be capable of sliding along the operation direction D2. In the housing 310, the biasing member 362 is disposed below the force transmission member 340, and the biasing member 362 biases the force transmission member 340 toward the upper side. One end portion of the biasing member 362 is fixed to the bottom surface of the member accommodating space 316 of the housing 310, and the protrusion 342 (FIG. 19) of the force transmission member 340 is inserted into a hollow portion at another end portion of the biasing member 362.

The force transmission member 340 is capable of transmitting to the through-member 330 a force along the operation direction D2 that causes the operating member 380 to approach the through-member 330. That is, as shown in FIGS. 15 and 16, when the force transmission member 340 is positioned in the retracted position P3 that is upwardly retracted, the protrusion 335 of the through-member 330 is positioned at a part on the lower proximal end side of the communication groove 345 of the force transmission member 340 (hereinafter referred to as "closed position portion 345A"), and as a result, the through-member 330 is placed in a position on the proximal end side, that is, in the non-pressing position P1 not pressing the hemostasis valve 120. The retracted position P3 of the force transmission member 340 is an example of a third position in the claims.

Furthermore, as shown in FIG. 17, when the force transmission member 340 receives a pressing force from the operating member 380 and moves from the retracted position P3 along the operation direction D2 to the advanced position P4 below, the protrusion 335 of the through-member 330 relatively moves to the upper distal end side inside the communication groove 345 of the force transmission member 340 and reaches a part on the upper distal end side (hereinafter referred to as "open position portion 345B"), and as a result, the through-member 330 is pressed and moves to a position on the distal end side, that is, to the pressing position P2 that presses and opens the hemostasis valve 120. The advanced position P4 of the force transmission member 340 is an example of a fourth position in the claims.

The holding mechanism 360 (FIG. 15) is a so-called double knock mechanism, and switches between a state in which the force transmission member 340 is held in the retracted position P3 and a state in which the force transmission member 340 is held in the advanced position P4 each time the operating member 380 slides along the operation direction D2. The holding mechanism 360 includes an outer cylinder 350 and a rotor 370. In the present embodiment, a portion of the operating member 380 and the biasing member 362 also constitute a portion of the holding mechanism 360.

Because the configuration of the holding mechanism 360 is the same as the configuration of the holding mechanism 160 of the opening/closing mechanism 100 of the first embodiment described above, the description will be omitted. That is, in the description of the configuration of the holding mechanism 160 of the opening/closing mechanism 100 above, the outer cylinder 150 may be interpreted as being the outer cylinder 350, the rotor 170 may be interpreted as being the rotor 370, and the biasing member 162 may be interpreted as being the biasing member 362. However, in the present embodiment, the biasing member 362 is not disposed between the force transmission member 340 and the rotor 370, and as mentioned above, is disposed between the housing 310 and the force transmission member 340. Even in such an arrangement, the biasing member 362 biases the force transmission member 340 along the operation direction D2 toward the external side of the housing 310.

### B-3. Motion of Opening/Closing Mechanism:

Next, the motion of the opening/closing mechanism 300 for the hemostasis valve 320 will be described. In the initial state, as shown in FIGS. 15 and 16, the holding mechanism 360 is in the rotor-retracted state. In this state, the force transmission member 340 is positioned in the retracted position P3, and the through-member 330 is not pressed toward the distal end side by the force transmission member 340 and is positioned in the non-pressing position P1. Therefore, the hemostasis valve 320 is closed, and the opening/closing mechanism 300 is the closed state opening/closing mechanism 300c.

For example, when the index finger of the operator that is gripping the medical connector 10A applies an operation (hereinafter, referred to as "valve-opening operation") of pressing the operating member 380 along the operation direction D2 to the inner side of the housing 310 (that is, in a downward direction), the operating member 380 slides downward along the operation direction D2. Accordingly, the holding mechanism 360 switches from the rotor-retracted state to the rotor-advanced state. In this state, as shown in FIG. 17, the force transmission member 340 is displaced to the advanced position P4, and the through-member 330 is pressed toward the distal end side by the force transmission member 340 and is displaced to the pressing position P2. As a result, the distal end portion of the through-member 330 presses the hemostasis valve 320, which causes the hemostasis valve 320 to be set to the open state, and the opening/closing mechanism 300 becomes the open state opening/closing mechanism 300o.

Furthermore, when the opening/closing mechanism 300 is the open state opening/closing mechanism 300o, for example, in the same manner as the valve-opening operation, when an operation (hereinafter, referred to as "valve-closing operation") of pressing the operating member 380 along the operation direction D2 to the inner side of the housing 310 (that is, in a downward direction) is applied, the operating member 380 slides downward along the operation direction D2. Accordingly, the holding mechanism 360 switches from the rotor-advanced state to the rotor-retracted state. In this state, as shown in FIGS. 15 and 16, the force transmission member 340 is displaced to the retracted position P3, and the through-member 330 is pressed toward the distal end side by the force transmission member 340 and returns to the non-pressing position P1. As a result, the through-member 330 does not press the hemostasis valve 320, which causes the hemostasis valve 320 to be set to the closed state, and the opening/closing mechanism 300 becomes the closed state opening/closing mechanism 300c.

In this way, the opening/closing mechanism 300 switches to the closed state opening/closing mechanism 300c or the open state opening/closing mechanism 300o each time the pressing operation (the valve-opening operation and the valve-closing operation) is performed with respect to the operating member 380.

### B-4. Technical Effects of Opening/Closing Mechanism

Because the opening/closing mechanism 300 of the second embodiment has the same configuration as the first embodiment described above, like the first embodiment, it is possible for an operator to very easily perform the opening and closing operation of the hemostasis valve 320 while gripping the medical connector 10A, and the operability of the opening/closing mechanism 300 can be improved.

The holding mechanism 360 includes a cylindrical biasing member 362 that biases the force transmission member 340 so as to approach the operating member 380, and the surface facing the biasing member 362 is formed having a protrusion 342 that is inserted into the hollow portion of the biasing member 362. Therefore, like the opening/closing mechanism 100 of the first embodiment, according to the opening/closing mechanism 300 of the second embodiment, it is possible to easily and accurately perform the positioning between the force transmission member 340 and the biasing member 362, and it is also possible to effectively transmit the biasing force of the biasing member 362 to the force transmission member 340, which enables the accuracy of the motion of the opening/closing mechanism 300 to be improved.

The angle formed between the extending direction (Z-axis direction) of the lumen 313 of the housing 310 and the operation direction D2 of the operating member 380 is substantially 90 degrees. Therefore, according to the opening/closing mechanism 300 of the second embodiment, it is possible for an operator to very easily perform the opening and closing operation of the hemostasis valve 320 with the thumb or index finger while gripping the medical connector 10A, and the operability of the opening/closing mechanism 300 can be effectively improved.

The through-member 330 has a protrusion 335 that protrudes in the left-right direction, which is a direction orthogonal to both the extending direction (Z-axis direction) of the lumen 313 of the housing 310 and the operation direction D2, and the force transmission member 340 is formed having a communication groove 345 into which the protrusion 335 is inserted. The communication groove 345 extends in a direction orthogonal to the left-right direction, and a direction that is not parallel to both the extending direction (Z-axis direction) of the lumen 313 of the housing 310 and the operation direction D2. Therefore, according to the opening/closing mechanism 300 of the second embodiment, the sliding of the force transmission member 340 along the up-down direction substantially orthogonal to the extending direction of the lumen 313 of the housing 310 and the sliding of the through-member 330 in the extending direction can be efficiently converted, and the operability of the opening/closing mechanism 300 can be effectively improved.

### B-5. Modification of Opening/Closing Mechanism

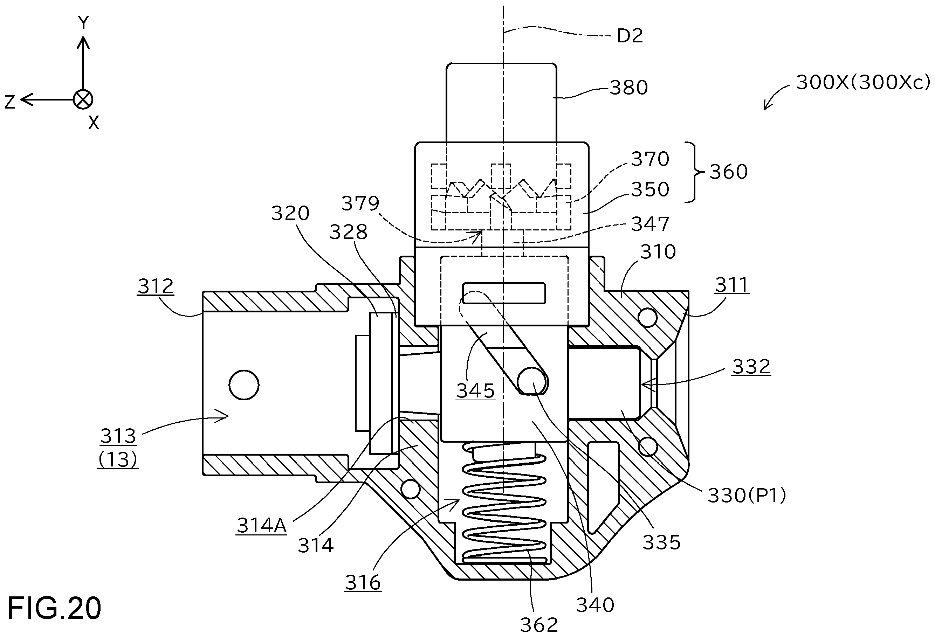

FIG. 20 is an explanatory diagram showing a configuration of a modification (opening/closing mechanism 300X) of the opening/closing mechanism 300 of the second embodiment. The opening/closing mechanism 300X of the modification shown in FIG. 20 differs from the opening/closing mechanism 300 of the second embodiment described above in that the operating member 380 and the force transmission member 340 are an integrated member. Specifically, a connection shaft 347 upwardly extends from the upper surface of the force transmission member 340, and the connection shaft 347 passes through the through-hole 379 formed in the rotor 370 up to the operating member 380, and is connected to the operating member 380. As a result, in the opening/closing mechanism 300X of the modification, the operating member 380 and the force transmission member 340 are integrated and slide along the operation direction D2. In the opening/closing mechanism 300X of such a modification, the holding mechanism 360 switches between a state in which the force transmission member 340 is held in the retracted position P3 and a state in which the force transmission member 340 is held in the advanced position P4 each time the operating member 380 slides along the operation direction D2. Therefore, according to the opening/closing mechanism 300X of the modification, the operability of the opening/closing mechanism 300 can be similarly improved.

### C. Third Embodiment:

### C-1. Configuration of Medical Connector:

FIG. 21 is an explanatory diagram illustrating a longitudinal sectional configuration (YZ section) of a medical connector 10B according to a third embodiment. Hereinafter, those components of the medical connector 10B of the third embodiment that are the same components as those of the medical connector 10 of the first embodiment described above are denoted by the same reference numerals and the description thereof is omitted as appropriate.

The medical connector 10B includes an opening/closing mechanism 100 and a fixing mechanism 200B. Because the opening/closing mechanism 100 included in the medical connector 10B is the same as the opening/closing mechanism 100 included in the medical connector 10 of the first embodiment, the description will be omitted, while the fixing mechanism 200B which is different from that of the first embodiment will be described.

### C-2. Configuration of Fixing Mechanism:

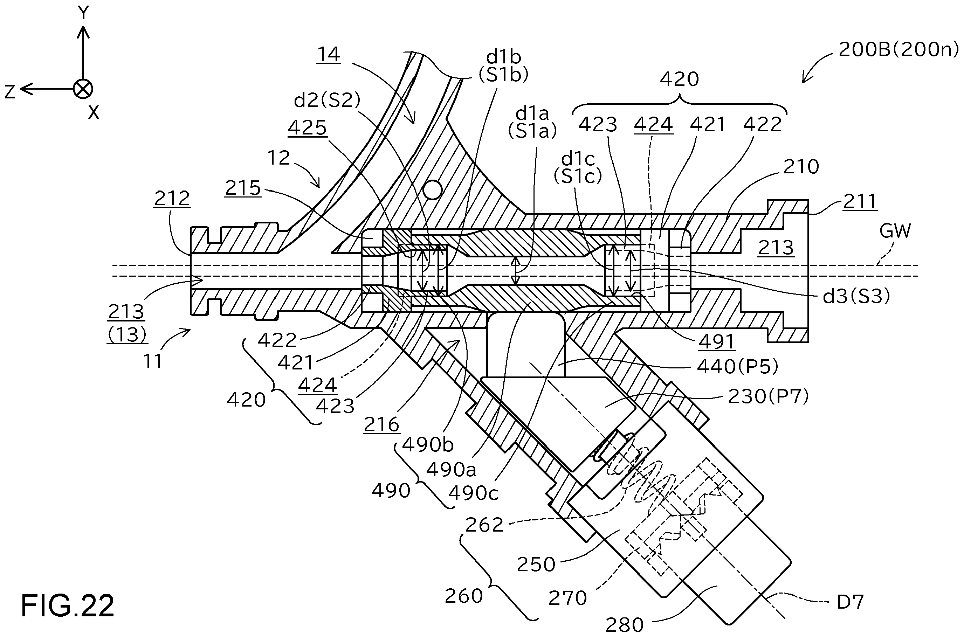

FIGS. 22 and 23 are explanatory diagrams illustrating the configuration of the fixing mechanism 200B, and the configuration in a longitudinal cross-section (YZ cross-section) is illustrated. FIG. 22 shows the fixing mechanism 200B in a state in which the fixing of a long medical device such as the guide wire GW has been released (released state fixing mechanism 200n), and FIG. 23 shows the fixing mechanism 200B in a state in which a long medical device has been fixed (fixed state fixing mechanism 200f). Note that some of the members are illustrated as a side view.

The fixing mechanism 200B of the third embodiment includes a cylindrical body 490 instead of the cylindrical body 290 of the fixing mechanism 200 of the first embodiment. The cylindrical body 490 is different from the cylindrical body 290 in the configuration of the through-hole 491. That is, the inner diameter of the through-hole 491 passing through the cylindrical body 490 in the longitudinal direction changes according to the position along the axial direction. More specifically, in the cylindrical body 490, the inner diameter d1a of the through-hole 491 at a center portion 490a is smaller than the inner diameter dib at a distal end portion 490b, and smaller than the inner diameter d1c at a proximal end portion 490c. In other words, the through-hole 491 has a larger diameter at the distal end portion and the proximal end portion than the center portion. Therefore, the area of the through-hole 491 in the transverse cross-section of the fixing mechanism 200B has the area S1a of the through-hole 491 at the transverse cross-section of the center portion 490a (more specifically, the transverse cross-section of the fixing mechanism 200B (the same applies below)) smaller than the area S1b at the distal end portion 490b and the area S1c at the proximal end portion 490c. The shape of the through-hole 491 in the transverse cross-section at each position may be any shape, such as a circular shape. The through-hole 491 is an example of a first through-hole in the claims.

Like the first embodiment, stoppers 420 are attached to the distal end portion and the proximal end portion of the cylindrical body 490. Each stopper 420 includes a main body portion 421 having substantially the same outer diameter as the center portion 490a of the cylindrical body 490, a first small diameter portion 423 that is positioned on the cylindrical body 490 side of the main body portion 421 and has a smaller outer diameter than the main body portion 421, and a second small diameter portion 422 that is positioned on the opposite side of the main body portion 421 to the cylindrical body 490 and has a smaller outer diameter than the main body portion 421. The first small diameter portion 423 is inserted into the through-hole 491. Furthermore, the main body portion 421 is formed having a recess 424 to which a protrusion (not illustrated) of the housing 210 is fitted. As a result of the fitting between the protrusion and the recess 424, the stoppers 420 are positioned in the front-rear direction with respect to the housing 210, and consequently, the cylindrical body 490 is positioned in the front-rear direction with respect to the housing 210. In other words, the stopper 420 on the distal end side connects the distal end portion 490b to the housing 210, and the stopper 420 on the proximal end side connects the proximal end portion 490c to the housing 210. Each stopper 420 is formed having a through-hole 425 that communicates with the through-hole 491. The shape of the through-hole 425 in the transverse cross-section at each portion may be any shape, such as a circular shape. The stopper 420 on the distal end side is an example of a distal end side connection portion in the claims, and the through-hole 425 of the stopper 420 on the distal end side is an example of a second through-hole in the claims. Furthermore, the stopper 420 on the proximal end side is an example of a proximal end side connection portion in the claims, and the through-hole 425 of the stopper 420 on the proximal end side is an example of a third through-hole in the claims.

In the first small diameter portion 423 of the stopper 420 on the distal end side (the part inserted inside the through-hole 491 of the cylindrical body 490), the inner diameter d2 of the through-hole 425 is larger than the inner diameter d1a of the through-hole 491 at the center portion 490a. Therefore, the area S2 of the through-hole 425 in the transverse cross-section of the first small diameter portion 423 of the stopper 420 on the distal end side is larger than the area S1a of the through-hole 491 in the transverse cross-section of the center portion 490a of the cylindrical body 490. Similarly, in the first small diameter portion 423 of the stopper 420 on the proximal end side (the part inserted inside the through-hole 491 of the cylindrical body 490), the inner diameter d3 of the through-hole 425 is larger than the inner diameter d1a of the through-hole 491 at the center portion 490a of the cylindrical body 490. Therefore, the area S3 of the through-hole 425 in the transverse cross-section of the first small diameter portion 423 of the stopper 420 on the proximal end side is larger than the area S1a of the through-hole 491 in the transverse cross-section of the center portion 490a of the cylindrical body 490.

In the present embodiment, the outer diameter of the center portion 490a is larger than the diameter of the distal end portion 490b, and larger than the outer diameter of the proximal end portion 490c. Therefore, in the cylindrical body 490 accommodated in the substantially hollow cylinder-shaped cylindrical body accommodating space 215 of the housing 210, the outer peripheral surface of the center portion 490a makes contact with, or is close to, the inner peripheral surface of the cylindrical body accommodating space 215, while the outer peripheral surfaces of the distal end portion 490b and the proximal end portion 490c are separated from the inner peripheral surface of the cylindrical body accommodating space 215.

The fixing mechanism 200B of the third embodiment includes a pressing member 440 instead of the pressing member 240 of the first embodiment. The pressing member 440 has a shape in which the part on the upper side of the through-hole 241 of the pressing member 240 of the first embodiment has been removed. That is, the pressing member 440 is disposed below the center portion 490a of the cylindrical body 490, and in the released state described above, the upper surface of the pressing member 440 makes contact with, or is close to, the outer peripheral surface of the center portion 490a of the cylindrical body 490.

Like the first embodiment, the pressing member 440 is capable of sliding along the up-down direction (Y-axis direction), which is a direction orthogonal to the axial direction (Z-axis direction) of the cylindrical body 490. The pressing member 440, which is capable of sliding in the up-down direction, can be positioned in the non-pressing position P5 shown in FIG. 22, which substantially does not press the cylindrical body 490, and the pressing position P6 shown in FIG. 23, which is upwardly displaced form the non-pressing position P5. Here, the state of substantially not pressing includes, in addition to a state where the upper surface of the pressing member 440 is separated from the outer peripheral surface of the cylindrical body 490, a state where the upper surface of the pressing member 440 is in close contact with the outer peripheral surface of the cylindrical body 490.

On the other hand, when the pressing member 440 is positioned in the pressing position P6 (FIG. 23), the pressing member 440 upwardly presses the center portion 490a of the cylindrical body 490 from the outer peripheral side and elastically deforms the center portion 490a. In this state, a contact position CP1, being a lower side part of the inner peripheral surface of the center portion 490a, and a contact position CP3, being an upper side part of the inner peripheral surface of the center portion 490a, which are on the inner peripheral surface of the through-hole 491 of the elastically deformed center portion 490a, are pressed against the long medical device, and the sliding of the long medical device in the front-rear direction is regulated as a result of the long medical device being fixed by the fixing mechanism 200B by being held between the two contact positions CP1 and CP3.

Here, as mentioned above, the area S2 of the through-hole 425 in the transverse cross-section of the first small diameter portion 423 of the stopper 420 on the distal end side and the area S3 of the through-hole 425 in the transverse cross-section of the first small diameter portion 423 of the stopper 420 on the proximal end side are larger than the area S1a of the through-hole 491 in the transverse cross-section of the center portion 490a of the cylindrical body 490. Therefore, even in a state in which the long medical device is fixed as a result of the pressing member 440 pressing the center portion 490a of the cylindrical body 490, the pressing of the long medical device against the inner peripheral surfaces of the stoppers 420 is suppressed, and damage to the long medical device can be more effectively suppressed. Note that such an effect can be obtained in a solid long medical device such as a guide wire, but can be especially obtained in a long medical device having a hollow portion such as a catheter.

### C. Modifications:

The technique disclosed herein is not limited to the embodiment described above, and various modifications can be made within a scope that does not depart from the gist thereof. For example, the following modifications can be made.

The configurations of the medical connector 10, the opening/closing mechanisms 100 and 300, and the fixing mechanism 200 in the embodiments described above are merely examples, and various modifications are possible. For example, in the first embodiment described above, a protrusion 142 that is inserted into the hollow portion of the biasing member 162 is formed on the surface of the force transmission member 140 facing the biasing member 162, but the protrusion 142 does not have to be formed. The protrusion 232 of the force transmission member 230 and the protrusion 342 of the force transmission member 340 can be similarly omitted.

In the embodiments described above, although a double knock mechanism is used as the holding mechanism 260 that switches between a state in which the force transmission member 230 is held in the retracted position P7 or the state in which the force transmission member 230 is held in the advanced position P8, another mechanism such as a heart cam mechanism may also be used as the holding mechanism 260. Similarly, another mechanism such as a heart cam mechanism may also be used as the holding mechanism 160 and/or the holding mechanism 360 instead of a double knock mechanism.

In the embodiments described above, the housing 210 and the stoppers 220 and 240 may be an integrated member.

The dimensions and materials of each member in the embodiments above are merely examples, and various modifications are possible.

In the embodiments described above, although the medical connector 10 includes both the opening/closing mechanism 100 (or the opening/closing mechanism 300) and the fixing mechanism 200, the medical connector 10 may include only one of the opening/closing mechanism 100 (or the opening/closing mechanism 300) and the fixing mechanism 200.

### DESCRIPTION OF REFERENCE NUMERALS

10: Medical connector
11: Main pipe portion
12: Branched pipe portion
13: Lumen
14: Lumen
20: Rotator
100: Opening/closing mechanism
110: Housing
110D: Distal end side housing part
110P: Proximal end side housing part
111: Proximal end side opening
112: Distal end side opening
113: Lumen
114: Partition wall
114A: Through-hole
115: Guide groove
116: Member accommodating space
119: Protrusion
120: Hemostasis valve
121: Slit
122: Through-hole
130: Through-member
131: Main body portion
132: Through-hole
133: Flange portion
134: Guide protrusion
135: Guide protrusion
140: Force transmission member
142: Protrusion
143: Distal end surface
144: Guide groove
145: Recess
147: Connection shaft
150: Outer cylinder
153: Outer cylinder end surface cam
154: Shallow groove portion
155: Deep groove portion
160: Holding mechanism
162: Biasing member
170: Rotor
173: Protrusion
174: Recess
179: Through-hole
180: Operating member
181: Sliding contactor
182: Operating member end surface cam
200: Fixing mechanism
210: Housing
211: Proximal end side opening
212: Distal end side opening
213: Lumen
214: Retraction space
215: Cylindrical body accommodating space
216: Member accommodating space
220: Stopper
221: Main body portion
222: Second small diameter portion
223: First small diameter portion
224: Recess
230: Force transmission member
232: Protrusion
233: Surface
234: Upper surface
235: Guide groove
240: Pressing member
241: Through-hole
244: Lower surface
245: Guide protrusion
250: Outer cylinder
260: Holding mechanism
262: Biasing member
270: Rotor
280: Operating member
290: Cylindrical body
291: Through-hole
300: Opening/closing mechanism
310: Housing
311: Proximal end side opening
312: Distal end side opening
313: Lumen
314: Partition wall
314A: Through-hole
316: Member accommodating space
320: Hemostasis valve
321: Slit
322: Through-hole
328: Boss
330: Through-member
332: Through-hole
335: Protrusion
340: Force transmission member
342: Protrusion
343: Protrusion
344: Through-hole
345: Communication groove
345A: Closed position portion
345B: Open position portion
347: Connection shaft
350: Outer cylinder
360: Holding mechanism
362: Biasing member
370: Rotor
379: Through-hole
380: Operating member
10B: Medical connector (third embodiment)
200B: Fixing mechanism
420: Stopper
421: Main body portion
422: Second small diameter portion
423: First small diameter portion
424: Recess
425: Through-hole
440: Pressing member
490: Cylindrical body
490a: Center portion
490b: Distal end portion
490c: Proximal end portion
491: Through-hole
CP1: Contact position
CP2: Contact position
CP3: Contact position
GC: Guiding catheter
GW: Guide wire

## Claims

1. An opening/closing mechanism for a hemostasis valve comprising:
a tubular housing formed having a lumen that communicates with a distal end side opening and a proximal end side opening;
a hemostasis valve attached inside the housing and normally in a closed state, which is switched to an open state when pressed from a proximal end side such that a through-hole is formed that communicates with the distal end side opening of the housing;
a through-member that is accommodated inside the housing so as to be capable of sliding along a first direction, being an extending direction of the lumen further toward a proximal end side than the hemostasis valve, and is formed having a through-hole that communicates with the proximal end side opening of the housing, the through-member being capable of being positioned in a first position that places the hemostasis valve in the closed state, and a second position, being a position further toward a distal end side along the first direction than the first position, which places the hemostasis valve in the open state by pressing the hemostasis valve, and communicates the through-hole of the hemostasis valve and the through-hole of the through-member;
an operating member that is capable of sliding along a second direction that is not parallel to the first direction;
a force transmission member that is disposed so as to be capable of sliding in the second direction between the through-member and the operating member, and that transmits, to the through-member, a force that moves the operating member along the second direction so as to approach the through-member, the force transmission member being capable of being positioned in a third position that positions the through-member in the first position, and a fourth position that presses the through-member and positions the through-member in the second position; and
a holding mechanism that switches to a state in which the force transmission member is held in the third position, or a state in which the force transmission member is held in the fourth position each time the operating member slides along the second direction.

2. The opening/closing mechanism for a hemostasis valve according to claim 1, wherein
the operating member and the force transmission member are an integrated member.

3. The opening/closing mechanism for a hemostasis valve according to claim 1 or 2, wherein
the holding mechanism comprises a cylindrical biasing member that directly biases, or biases via another member, the force transmission member along the second direction toward an external side of the housing, and
a surface of the force transmission member or of the another member facing the biasing member is formed having a protrusion that is inserted into a hollow portion of the biasing member.

4. The opening/closing mechanism for a hemostasis valve according to any one of claims 1 to 3, wherein
an angle formed between the first direction and the second direction is 35 degrees or more and 55 degrees or less.

5. The opening/closing mechanism for a hemostasis valve according to claim 4, wherein
the through-member comprises a main body portion that is formed having a through-hole, and a flange portion that protrudes from the main body portion in a third direction, which is orthogonal to the first direction, and
the force transmission member makes contact with a surface of the flange portion on a proximal end side, while also being capable of a relative sliding movement with respect to the through-member along the third direction.

6. The opening/closing mechanism for a hemostasis valve according to any one of claims 1 to 3, wherein
an angle formed between the first direction and the second direction is substantially 90 degrees.

7. The opening/closing mechanism for a hemostasis valve according to claim 6, wherein
the force transmission member is capable of sliding in the second direction,
the through-member comprises a protrusion that protrudes in a fourth direction, which is orthogonal to both the first direction and the second direction, and
the force transmission member is formed having a groove into which the protrusion is inserted, and which extends in a direction that is orthogonal to the fourth direction and not parallel to both the first direction and the second direction.

8. A fixing mechanism for a long medical device, comprising:
a tubular housing formed having a lumen that communicates with a distal end side opening and a proximal end side opening;
a flexible cylindrical body attached inside the housing and formed having a through-hole into which the long medical device is inserted, the through-hole communicating with the distal end side opening and the proximal end side opening of the housing;
a pressing member that is accommodated inside the housing and capable of sliding along a sixth direction, which is a direction orthogonal to a fifth direction that is parallel to an axis of the cylindrical body, the pressing member being capable of being positioned in a fifth position, and a sixth position that is displaced from the fifth position along the sixth direction, at which a part of the cylindrical body excluding both end portions is deformed by being pressed from an outer peripheral side;
an operating member that is capable of sliding along a seventh direction that is not parallel to the fifth direction;
a force transmission member that is disposed so as to be capable of sliding in the seventh direction between the pressing member and the operating member, and that transmits, to the pressing member, a force that moves the operating member along the seventh direction so as to approach the pressing member, the force transmission member being capable of being positioned in a seventh position that positions the pressing member in the fifth position, and an eighth position that presses the pressing member and positions the pressing member in the sixth position; and
a holding mechanism that switches to a state in which the force transmission member is held in the seventh position, or a state in which the force transmission member is held in the eighth position each time the operating member slides along the seventh direction; wherein
when the pressing member is positioned in the sixth position, an inner peripheral surface of the deformed cylindrical body is pressed against the long medical device, which causes the long medical device to become fixed by the cylindrical body.

9. The fixing mechanism for a long medical device according to claim 8, wherein
the holding mechanism comprises a cylindrical biasing member that biases the force transmission member in a direction approaching the pressing member, and
a surface of the force transmission member facing the biasing member is formed having a protrusion that is inserted into a hollow portion of the biasing member.

10. The fixing mechanism for a long medical device according to claim 8 or 9, wherein
an angle formed between the fifth direction and the seventh direction is 35 degrees or more and 55 degrees or less.

11. The fixing mechanism for a long medical device according to claim 10, wherein
the force transmission member makes contact with a surface of the pressing member, while also being capable of a relative sliding movement with respect to the pressing member along the fifth direction.

12. A fixing mechanism for a long medical device, comprising:
a tubular housing formed having a lumen that communicates with a distal end side opening and a proximal end side opening, and which is capable of accommodating the long medical device in the lumen;
a flexible cylindrical body attached inside the housing and formed having a first through-hole into which the long medical device is inserted, the first through-hole communicating with the distal end side opening and the proximal end side opening of the housing;
a distal end side connection portion that connects a distal end portion of the cylindrical body to the housing, and is formed having a second through-hole that communicates with the first through-hole of the cylindrical body;
a proximal end side connection portion that connects a proximal end portion of the cylindrical body to the housing, and is formed having a third through-hole that communicates with the first through-hole of the cylindrical body; and
a pressing member that presses a pressed part of the cylindrical body excluding both end portions from a direction that is not parallel to an axis of the cylindrical body, and which is accommodated inside the housing; wherein
an area of the second through-hole in a transverse cross-section of the fixing mechanism of the distal end side connection portion and an area of the third through-hole in the transverse cross-section of the proximal end side connection portion are larger than an area of the first through-hole in the transverse cross-section of the pressed part.

13. The fixing mechanism for a long medical device according to claim 12, wherein
the pressing member is accommodated inside the housing and capable of sliding along a sixth direction, which is a direction orthogonal to a fifth direction that is parallel to an axis of the cylindrical body, and is capable of being positioned in a fifth position, and a sixth position that is displaced from the fifth position along the sixth direction, at which a part of the cylindrical body excluding both end portions is deformed by being pressed from an outer peripheral side, and
the fixing mechanism further comprises
an operating member that is capable of sliding along a seventh direction that is not parallel to the fifth direction,
a force transmission member that is disposed so as to be capable of sliding in the seventh direction between the pressing member and the operating member, and that transmits, to the pressing member, a force that moves the operating member along the seventh direction so as to approach the pressing member, the force transmission member being capable of being positioned in a seventh position that positions the pressing member in the fifth position, and an eighth position that presses the pressing member and positions the pressing member in the sixth position, and
a holding mechanism that switches to a state in which the force transmission member is held in the seventh position, or a state in which the force transmission member is held in the eighth position each time the operating member slides along the seventh direction, and
when the pressing member is positioned in the sixth position, an inner peripheral surface of the deformed cylindrical body is pressed against the long medical device, which causes the long medical device to become fixed by the cylindrical body.

14. The fixing mechanism for a long medical device according to claim 13, wherein
the holding mechanism comprises a cylindrical biasing member that biases the force transmission member in a direction approaching the pressing member, and
a surface of the force transmission member facing the biasing member is formed having a protrusion that is inserted into a hollow portion of the biasing member.

15. The fixing mechanism for a long medical device according to claim 13 or 14, wherein
an angle formed between the fifth direction and the seventh direction is 35 degrees or more and 55 degrees or less.

16. The fixing mechanism for a long medical device according to claim 15, wherein
the force transmission member makes contact with a surface of the pressing member, while also being capable of a relative sliding movement with respect to the pressing member along the fifth direction.

17. A medical connector comprising:
the opening/closing mechanism for a hemostasis valve according to any one of claims 1 to 7; and
the fixing mechanism for a long medical device according to any one of claims 8 to 16.
